# EUROPEAN PATENT SPECIFICATION

(11) **EP 2 334 808 B1**
(45) Date of publication and mention of the grant of the patent: **15.11.2017**
(21) Application number: 09787323.6
(22) Date of filing: 29.09.2009
(51) Int. Cl.: C12Q 1/68

(54) **METHOD FOR TESTING AND QUALITY CONTROLLING OF NUCLEIC ACIDS ON A SUPPORT**
VERFAHREN ZUR QUALITÄTSKONTROLLE VON NUKLEINSÄUREN AUF EINEM TRÄGER
PROCÉDÉ DE CONTROLE DE QUALITÉ DES ACIDES NUCLEIQUES SUR UN SUPPORT

(30) Priority: 01.10.2008 EP 08165577
(43) Date of publication of application: 22.06.2011
(73) Proprietor: Koninklijke Philips N.V., 5656 AE Eindhoven (NL)
(72) Inventor: PIERIK, Anke, NL-5656 AE Eindhoven (NL); STAPERT, Hendrik, R., NL-5656 AE Eindhoven (NL)
(74) Representative: Kroeze, Johannes Antonius
(86) International application number: PCT/IB2009/054259
(87) International publication number: WO 2010/038191

(56) References cited:
- WO-A1-89/11548
- WO-A2-2004/007684
- GB-B- 2 355 716
- US-A1- 2006 216 816
- YANG A X ET AL: "Analysis of the quality of contact pin fabricated oligonucleotide microarrays" MOLECULAR BIOTECHNOLOGY NOVEMBER 2006 HUMANA PRESS INC. US, vol. 34, no. 3, November 2006 (2006-11), pages 303-315, XP002561461
- SAIKI R K ET AL: "GENETIC ANALYSIS OF AMPLIFIED DNA WITH IMMOBILIZED SEQUENCE- SPECIFIC OLIGONUCLEOTIDE PROBES" PROCEEDINGS OF THE NATIONAL ACADEMY OF SCIENCES OF USA, NATIONAL ACADEMY OF SCIENCE, WASHINGTON, DC, US, vol. 86, no. 16, 1 August 1989 (1989-08-01), pages 6230-6234, XP000268602 ISSN: 0027-8424
- LAUSTED CHRISTOPHER ET AL: "POSaM: a fast, flexible, open-source, inkjet oligonucleotide synthesizer and microarrayer" GENOME BIOLOGY, BIOMED CENTRAL LTD., LONDON, GB, vol. 5, no. 8, 27 July 2004 (2004-07-27), page R58, XP021012917 ISSN: 1465-6906
- PENCHOVSKY R ET AL: "END-SPECIFIC COVALENT PHOTO-DEPENDENT IMMOBILISATION OF SYNTHETIC DNA TO PARAMAGNETIC BEADS", NUCLEIC ACIDS RESEARCH, INFORMATION RETRIEVAL LTD, GB, vol. 28, no. 22, 1 January 2000 (2000-01-01), pages 1-06, XP001127053, ISSN: 0305-1048, DOI: 10.1093/NAR/28.22.E98
- Gali Steinberg ET AL: "Strategies for covalent attachment of DNA to beads", Biopolymers, vol. 73, no. 5, 5 April 2004 (2004-04-05), pages 597-605, XP055278299, US ISSN: 0006-3525, DOI: 10.1002/bip.20006

## Description

### FIELD OF THE INVENTION

The present invention relates to a method for testing nucleic acids on a support, comprising the immobilizition of one or more nucleic acids on a solid support allowing for an accurate and trackable positioning of nucleic acids on the support material via crosslinking by heat or light or via chemical immobilization, wherein each of the immobilized nucleic acids includes a stretch of nucleotides of only one basetype, the provision of a labeled oligonucleotide complementary to the stretch of nucleotides of only one basetype, wherein said labeled oligonucleotide is capable of forming a complex with each of the immobilized nucleic acids at the stretch of nucleotides of only one basetype; and the determination of a value indicative for the interaction between said nucleic acid and the complementary oligonucleotide via the amount of labeled oligonucleotide being in complex with the immobilized nucleic acid. The present invention further relates to a kit for testing nucleic acids on a support, comprising an array of nucleic acids immobilized on a solid support allowing for an accurate and trackable positioning of nucleic acids on the support material via crosslinking by heat or light or via chemical immobilization, wherein each of the immobilized nucleic acids includes a stretch of nucleotides of only one basetype, and a labeled oligonucleotide complementary to the stretch of nucleotides of only one basetype, wherein said labeled oligonucleotide is capable of forming a complex with each of the immobilized nucleic acids at the stretch of nucleotides of only one basetype. The invention additionally concerns the use of a labeled oligonucleotide complementary to a stretch of nucleotides of only one basetype for testing the interaction between the complementary oligonucleotide and nucleic acids immobilized on a solid support allowing for an accurate and trackable positioning of nucleic acids on the support material via crosslinking by heat or light or via chemical immobilization, wherein each of the immobilized nucleic acids includes a stretch of nucleotides of only one basetype and wherein said labeled oligonucleotide is capable of forming a complex with each of the immobilized nucleic acids at said stretch of nucleotides of only one basetype.

### BACKGROUND OF THE INVENTION

Biochips or biological microarrays, in particular DNA microarrays, have become an important tool in modern molecular biology and medicine. Typically the chips consist of an arrayed series of a large number of microscopic spots of nucleic acid molecules, each containing small amounts of a specific nucleic acid sequence. This can be, for example, a short section of a gene or other DNA element that are used as capture probes to hybridize a cDNA or cRNA sample (a target or target probe) under conditions, which allow a binding between the capture probe and the corresponding target. Capture probe-target hybridization is typically detected and quantified by fluorescence-based detection of fluorophore-labeled targets to determine relative abundance of nucleic acid sequences in the target.

Microarray technology evolved from Southern blotting, where fragmented DNA is attached to a substrate and then probed with a known gene or fragment. The use of a collection of distinct DNAs in arrays for expression profiling was first described in 1987, and the arrayed DNAs were used to identify genes whose expression is modulated by interferon. These early gene arrays were made by spotting cDNAs onto filter paper with a pin-spotting device. The use of miniaturized microarrays, in particular for gene expression profiling was first reported in the 1990s. A complete eukaryotic genome on a microarray was published in 1997.

Nucleic acid oligomer probes have long been used to detect complementary nucleic acid sequences in a nucleic acid sequence of interest or target probe and have been employed in order to detect expression of particular genes, e.g. in Northern blots. In a microarray format, the oligonucleotide probe is immobilized on a solid support. Arrays of oligonucleotides prepared in such a way can be used to detect complementary target nucleic acid sequences, as has been described in WO 89/10977 and WO 89/11548. In oligonucleotide microarrays, the probes are typically short sequences designed to match parts of the sequence of known or predicted open reading frames.

A variety of technologies may be used in order to fabricate such microarrays. The techniques include printing with fine-pointed pins, photolithography using pre-made masks, photolithography using dynamic micromirror devices, ink-jet printing (Lausted C et al., 2004, Genome Biology 5: R58), or electrochemistry. Typically, the capture probes are attached to a solid surface by a covalent bond to a chemical matrix. As an example, such a solid surface may have the form of microscopic beads.

The photolithographic technique is directed to the production of oligonucleotide arrays by synthesizing the sequences directly onto the array surface. The technique involves photolithographic synthesis on a silica substrate where light and light-sensitive masking agents are utilized to generate a sequence one nucleotide at a time across the entire array (Pease et al., 1994, PNAS 91: 5022-5026). Each applicable probe is selectively unmasked prior to bathing the array in a solution of a single nucleotide, then a masking reaction takes place and the next set of probes are unmasked in preparation for a different nucleotide exposure. After several repetitions, the sequences of every probe become fully constructed. Accordingly constructed oligonucleotides maybe longer (e.g. 60-mers) or shorter (e.g. 25-mers) depending on the desired purpose.

In spotted microarrays, the oligonucleotide probes are deposited as intact sequences, i.e. the probes are synthesized prior to deposition on the array surface and are then spotted onto the substrate. A common approach utilizes an array of fine pins or needles controlled by a robotic arm that is dipped into wells containing DNA probes and then depositing each probe at designated locations on the array surface, or an ink jet printing device, which deposits the probe material via the ejection of droplets. The resulting array of probes represents the nucleic acid profiles of the prepared capture probes and can interact with complementary cDNA or cRNA target probes, e.g. derived from experimental or clinical samples. In addition, these arrays may be easily customized for specific experiments, since the probes and printing locations on the arrays can be chosen specifically.

During the manufacturing process of microarrays it is necessary to know whether each spot on the substrate is present and can still hybridize.

The control, adjustment and fine-tuning of spotting and deposition processes for the production of microarrays has been described, for example, in GB 2355716.

However, this approach is focused on the printing process itself and involves the detection of vibrations in the ink jet device or of equipment malfunctions, but provides no solution which would guarantee the correct and efficient deposition of the droplets on the substrate or which would allow to control the fate of the droplets after the printing process is terminated. Such a printing control method cannot be used for the evaluation of the quality of the spotted probes. In particular it cannot ascertain whether the deposited nucleic acid capture probes are indeed present or capable of hybridizing with target molecules.

Yang, A. X., et al. describe the analysis of quality of contact pin fabricated oligonucleotide microarrays; Molecular Biotechnology, Vol. 34, 2006, pages 303-315.

The US 2006/0216816 A1 concerns a flow path system for use in the detection of hybridization, and includes a capillary provided at a predetermined location thereof with a reaction section packed with beads. On the beads, a linker having a sequence of bases of the same type is immobilized, and a target nucleic acid complementarily bound to the sequences of the bases of the same type is held.

In consequence, there is a need for a method which allows testing the condition and quality of nucleic acids deposited on a support.

### OBJECTS AND SUMMARY OF THE INVENTION

The present invention addresses this need and provides means and methods which allow the immobilization and subsequent testing and quality-controlling of nucleic acids deposited on a support.

Thus, one objective of the present invention is to provide means and methods, which permits to control the fate of spotted nucleic acids after the printing process is terminated and to evaluate the quality of the spotted probes.

The above objective is accomplished by a method for testing nucleic acids on a support which comprises the immobilization of one or more nucleic acids on a solid support via crosslinking by heat or light or via chemical immobilization, wherein each of the immobilized nucleic acids includes a stretch of nucleotides of only one basetype, the provision of a labeled oligonucleotide complementary to the stretch of nucleotides of only one basetype, wherein said labeled oligonucleotide is capable of forming a complex with each of the immobilized nucleic acids at the stretch of nucleotides of only one basetype, and the determination of a value indicative for the interaction between said nucleic acid and the complementary oligonucleotide via the amount of labeled oligonucleotide being in complex with the immobilized nucleic acid.

It is an advantage of the method according to the present invention that a cheap, versatile and universal tool is provided which allows the control and inspection of deposited nucleic acids in a simple and reliable interaction scheme. The method relies on the presence and use of capture probe nucleic acids which include a stretch of nucleotides of only one basetype. These nucleic acids provide a means for efficient immobilization on a substrate via said stretch of nucleotides of only one basetype, preferably via crosslinking by heat or light and, as further and independent feature, allow for an equal and uniform interaction between the stretch of nucleotides of only one basetype and a complementary oligonucleotide. Thus, only one oligonucleotide type is necessary, which is easier to synthesize than oligonucleotides composed of nucleotides of more than one basetype and reduces the overall costs of the quality control scheme. Since the oligonucleotides can be rather short, a further reduction of costs is feasible. A main advantage of the present invention thus lies in the possibility to directly and easily scrutinize the outcome of spotting and immobilization processes, in particular to check whether spots are entirely missing, whether molecules have not properly been immobilized due to, e.g. a natural degradation of the surface of the substrate over time, the omission of the application of an immobilization step or the degradation or modification of DNA so that it is no longer able to hybridize. Furthermore, the method is non-disruptive and allows the control of the condition of the deposited nucleic acids on the substrate, while the yield of the manufacturing process is not affected. In a preferred embodiment it is additionally possible to reuse the test oligonucleotides after a washing step for subsequent control reactions. Such a course of action additionally contributes to a limitation of costs and time within the context of quality control of deposited nucleic acids.

In a specific aspect of the present invention a kit for testing nucleic acids on a support is provided. Said kit comprises an array of nucleic acids immobilized on a solid support via crosslinking by heat or light or via chemical immobilization, wherein each of the immobilized nucleic acids includes a stretch of nucleotides of only one basetype and a labeled oligonucleotide complementary to the stretch of nucleotides of only one basetype, wherein said labeled oligonucleotide is capable of forming a complex with each of the immobilized nucleic acids at the stretch of nucleotides of only one basetype.

In a further aspect the present invention relates to the use of a labeled oligonucleotide complementary to a stretch of nucleotides of only one basetype for testing the interaction between the complementary oligonucleotide and nucleic acids immobilized on a solid support via crosslinking by heat or light or via chemical immobilization, wherein each of the immobilized nucleic acids includes a stretch of nucleotides of only one basetype and wherein said labeled oligonucleotide is capable of forming a complex with each of the immobilized nucleic acids at said stretch of nucleotides of only one basetype.

In a preferred embodiment of the present invention the testing of the interaction between the complementary oligonucleotide and nucleic acids comprises the determination of a value indicative for the amount of labeled oligonucleotide being in complex with said immobilized nucleic acid.

In a further preferred embodiment of the present invention the nucleic acid to be tested is a single-stranded DNA, RNA, PNA, CNA, HNA, LNA or ANA, an oligonucleotide thereof or any combination thereof.

In a further preferred embodiment of the present invention, said stretch of nucleotides of only one basetype included in the nucleic acids to be tested as mentioned above is a stretch of thymines, uracils or guanines.

In another preferred embodiment of the present invention, said stretch of nucleotides of only one basetype as mentioned above has a length from 2 to 100 nucleotides. In a further particularly preferred embodiment said stretch of nucleotides of only one basetype has a length of 16 nucleotides.

In yet another preferred embodiment of the present invention said crosslinking used for the immobilization of one or more nucleic acids on a solid support as mentioned above is a crosslinking by light performed at a wavelength of 200-300 nm. In a particularly preferred embodiment, said crosslinking by light is performed at a wavelength of 254 nm.

In another preferred embodiment of the present invention said crosslinking used for the immobilization of one or more nucleic acids on a solid support as mentioned above is a crosslinking by light performed at a wavelength of 300-500 nm. In a particularly preferred embodiment, said crosslinking by light is performed at a wavelength of 365 nm.

In a further preferred embodiment of the present invention said crosslinking used for the immobilization of one or more nucleic acids on a solid support as mentioned above is a crosslinking by light performed at a wavelength of 200-300 nm or 300-500 nm, which is carried out by using an amount of energy ranging from 0.1 Joule/cm² to 10 Joule/cm².

In a further preferred embodiment of the present invention said chemical immobilization used for the immobilization of one or more nucleic acids on a solid support mentioned above is a coupling between an amine-modified nucleic acid and a corresponding functional group on the solid support. In a particularly preferred embodiment said functional group is an epoxy, aldehyde, carboxylate or NHS group.

In yet another preferred embodiment of the present invention said stretch of nucleotides of only one basetype as mentioned above is located at the 3' or 5' terminus of said nucleic acid.

In a further preferred embodiment of the present invention the nucleic acid to be immobilized according to the invention is represented by the following formula:

5'-Yₙ-Xₘ-Bᵣ-Xₚ-Z_{q}-3'

with Y and Z being stretches of nucleotides of only one basetype, wherein Y and Z can be of the same or of a different basetype; X being a spacer, preferably composed of abasic nucleotides; B being a sequence of more than one basetype and n, m, r, p and q being the numbers of nucleotides in the nucleic acid, wherein the following conditions may apply: n, m, p, q, r >1; n, m, r > 1 and p, q = 0; p, q, r >1 and n, m = 0; n, q, r > 1 and m, p = 0; n, r > 1 and m, p, q = 0; q, r > 1 and n, m, p = 0.

In a further preferred embodiment of the present invention said labeled oligonucleotide as mentioned above comprises a fluorescent, radioactive or chemiluminescent label.

In another preferred embodiment of the present invention said solid support as mentioned above comprises amine-functionalized groups. In a particularly preferred embodiment of the present invention, said amine-functionalized groups comprising support comprises primary or secondary amines.

In a further particularly preferred embodiment of the present invention said amine-functionalized groups comprising support comprises a porous substrate. In an even more preferred embodiment said above mentioned porous substrate is composed of nylon.

In a further particularly preferred embodiment of the present invention said amine-functionalized groups comprising support comprises a non-porous substrate. In an even more preferred embodiment of the present invention said above mentioned non-porous substrate is composed of glass, poly-L-lysine coated material, nitrocellulose, polystyrene, cyclic olefin copolymer (COC), cyclic olefin polymer (COP), polypropylene, polyethylene or polycarbonate.

In a further preferred embodiment of the present invention said labeled oligonucleotide complementary to the stretch of nucleotides of only one basetype of the above mentioned method in accordance with the present invention is obtained for re-use in a further method step by increasing the temperature above the melting temperature of said labeled oligonucleotide.

In a further aspect the present invention relates to a method for analyzing nucleic acids, comprising the steps of: (a) immobilizing one or more nucleic acids on a solid support via crosslinking by heat or light or via chemical immobilization, wherein each of the immobilized nucleic acids includes a stretch of nucleotides of only one basetype; (b) providing a labeled oligonucleotide complementary to the stretch of nucleotides of only one basetype, wherein said labeled oligonucleotide is capable of forming a complex with each of the immobilized nucleic acids at the stretch of nucleotides of only one basetype; (c) detecting the presence of a specific sequence complementary to the sequence outside the stretch of nucleotides of only one basetype; and (d) determining a value indicative for the interaction between said nucleic acid and the complementary oligonucleotide via the amount of labeled oligonucleotide complementary to the stretch of nucleotides of only one basetype being in complex with the immobilized nucleic acids. Preferably, steps (b) and (c) are carried out simultaneously.

In a still another preferred embodiment of the present invention, the quality of nucleic acids immobilized in accordance with the present invention may additionally be tested in a method comprising the additional steps of (i) providing at least one labeled test oligonucleotide complementary to a predefined specific stretch of nucleotides outside the stretch of nucleotides of only one basetype, wherein said labeled oligonucleotide is capable of distinctively forming a complex with immobilized nucleic acids which comprise said specific stretch of nucleotides; and (ii) determining a value indicative for the interaction between said nucleic acid and the complementary test oligonucleotide via the presence of said test oligonucleotide being in complex with the predefined specific stretch of nucleotides outside the stretch of nucleotides of only one basetype of the immobilized nucleic acids which comprise said specific stretch of nucleotides.

In a still another preferred embodiment of the present invention the kit for testing nucleic acids on a support as mentioned above additionally comprises at least one labeled test oligonucleotide complementary to a predefined specific stretch of nucleotides outside the stretch of nucleotides of only one basetype, wherein said labeled oligonucleotide is capable of distinctively forming a complex with immobilized nucleic acids which comprise said specific stretch of nucleotides.

In yet another preferred embodiment of the present invention in said method for analyzing nucleic acids, in said method for determining the quality of nucleic acids immobilized in accordance with the present invention and in said kit for testing nucleic acids on a support as mentioned above, said labeled test oligonucleotide complementary to a predefined stretch of nucleotides outside the stretch of nucleotides of only one basetype is labeled with a label which is optically or chemically distinguishable from the label of the labeled oligonucleotide complementary to the stretch of nucleotides of only one basetype.

These and other characteristics, features and objectives of the present invention will become apparent from the following detailed description, taken in conjunction with the accompanying figures and examples, which demonstrate by way of illustration the principles of the invention. The description is given for the sake of example only, without limiting the scope of the invention.

### DESCRIPTON OF THE FIGURES

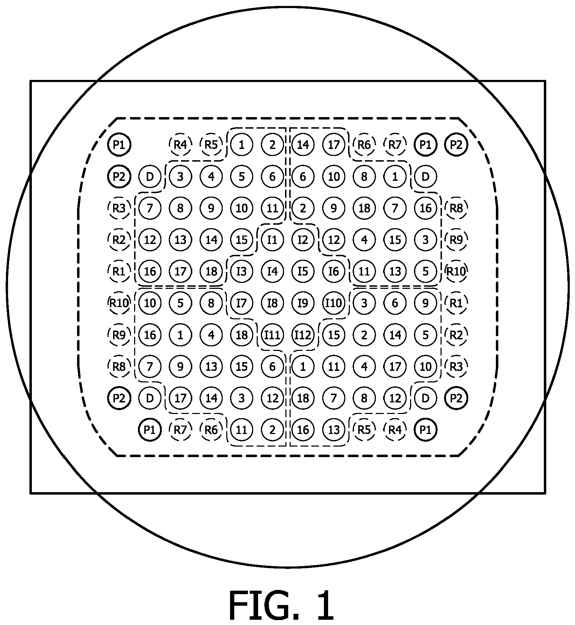

Fig. 1 shows a sample of a dot pattern as used for experiments described in the examples.

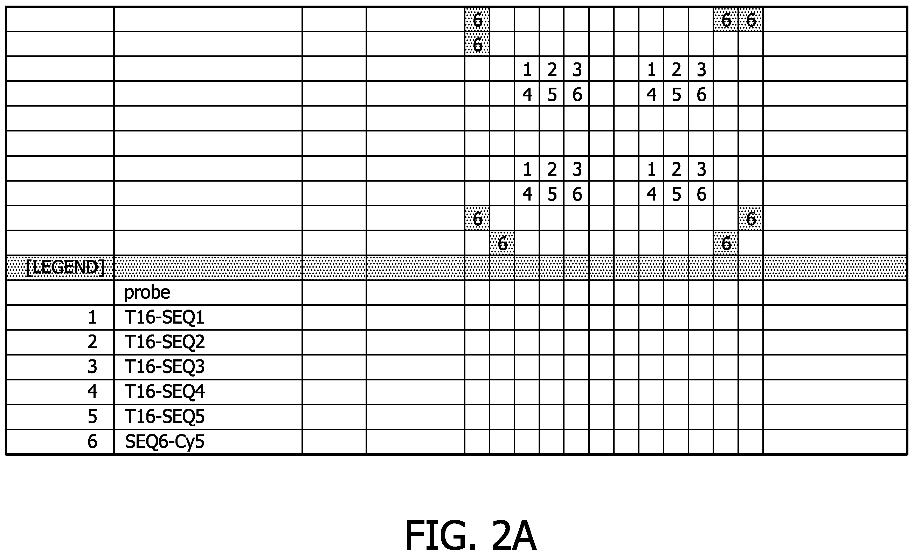

Fig. 2A depicts a membrane layout. Spots 1 to 5 (references number 1 to 5) represent unlabeled hybridization spot. These spots all contain a tail of 16 thymines (T16), but the hybridization part of the oligo contains a different sequence for every spot. Spot 6 (references number 6) contains an oligonucleotide with a fluorescent label. This oligonucleotide is used for gridding and calibration.

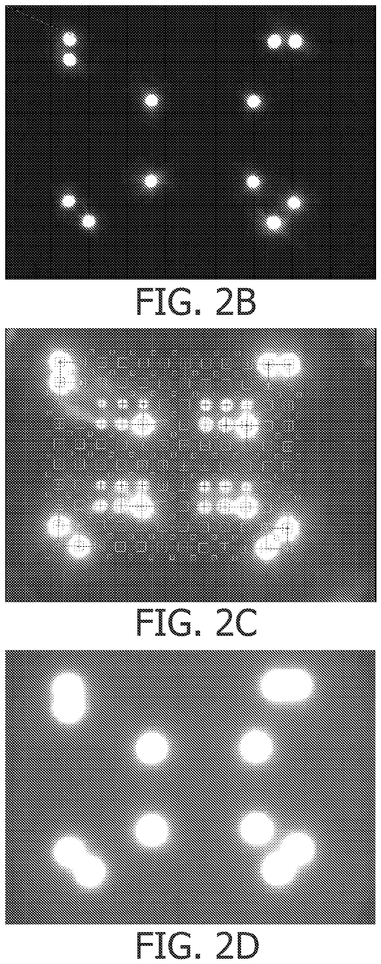

Fig. 2B depicts an image of the membrane whose layout is shown in Fig. 2A before hybridization. The image shows only signals on those spots which contain oligonucleotides with a fluorescent label (reference number 6) as indicated in Fig. 2A.
Fig. 2C depicts an image of the membrane shown in Fig. 2B after hybridization with a control probe. The membrane was incubated with a labeled A16 oligonucleotide. The hybridization spots are clearly visible. Signals on spots which contain oligonucleotides with a fluorescent label (reference number 6) as indicated in Fig. 2A are additionally visible as larger dots.
Fig. 2D depicts an image of the membrane shown in Fig. 2C directly after heating up in order to remove the control oligonucleotides from the capture probe spots. The hybridization spots, which were visible in Fig. 2C, do not show any signal anymore. The image still shows signals on those spots which contain oligonucleotides with a fluorescent label (reference number 6) as indicated in Fig. 2A.

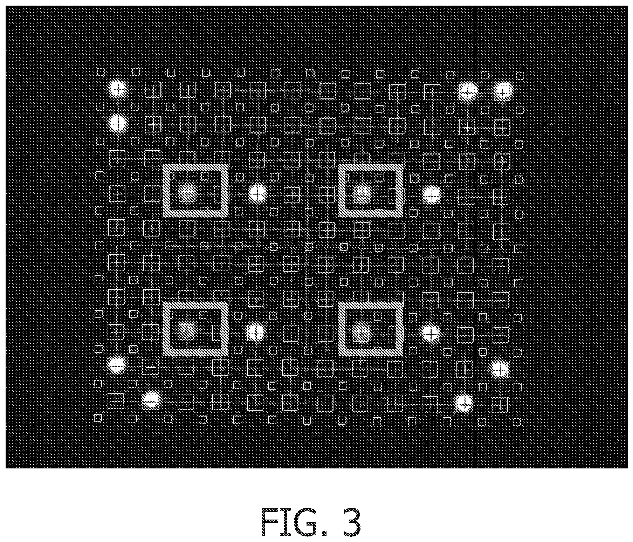

Fig. 3 depicts an image of a membrane after hybridization with a labeled antisense oligonucleotide complementary to the hybridization part of the capture probe on spot number 4 (reference number 4 as indicated in Fig. 2A). The membrane was used before in a control and test hybridization approach as depicted in Figs. 2B to 2D. Marked in thick squares are the spots which show a signal after hybridization with the labeled antisense oligonucleotide, corresponding to spot number 4 as indicated in Fig. 2A. The image demonstrates that the capture probe on the membrane was not damaged during the quality control step and can still be bound by an antisense oligonucleotide.

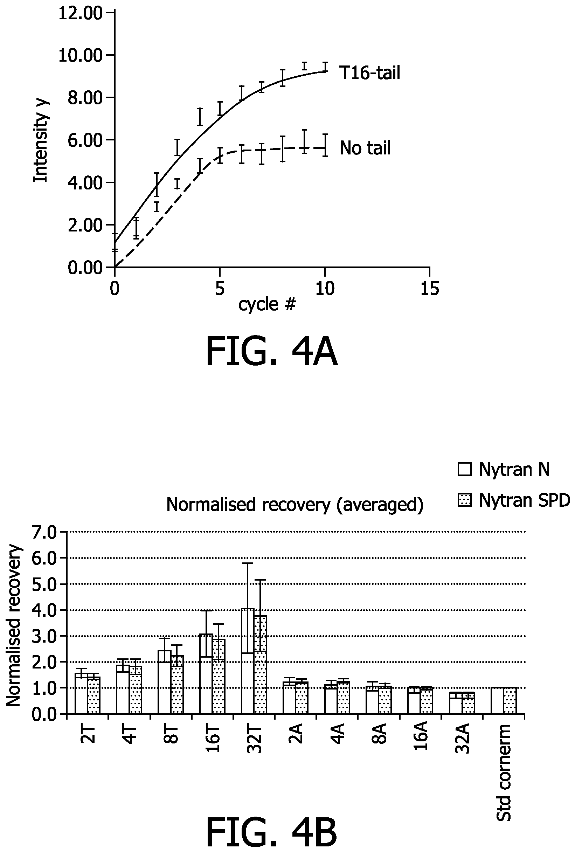

Fig. 4A depicts a real-time hybridization curve of capture oligonucleotides with no T-tail and with a T16-tail, i.e. comprising a stretch of 16 thymidines. The oligonucleotides comprising a T16-tail show increased hybridization signals, which are attributed to a higher recovery.
Fig. 4B shows normalized recoveries of deposited capture oligonucleotides comprising T or A nucleotides as a function of the base type (T or A) and the number of bases (2, 4, 8, 16 or 32). It is demonstrated that the recovery can be 3-4 fold increased when the number of T's increases from 2 to 32.

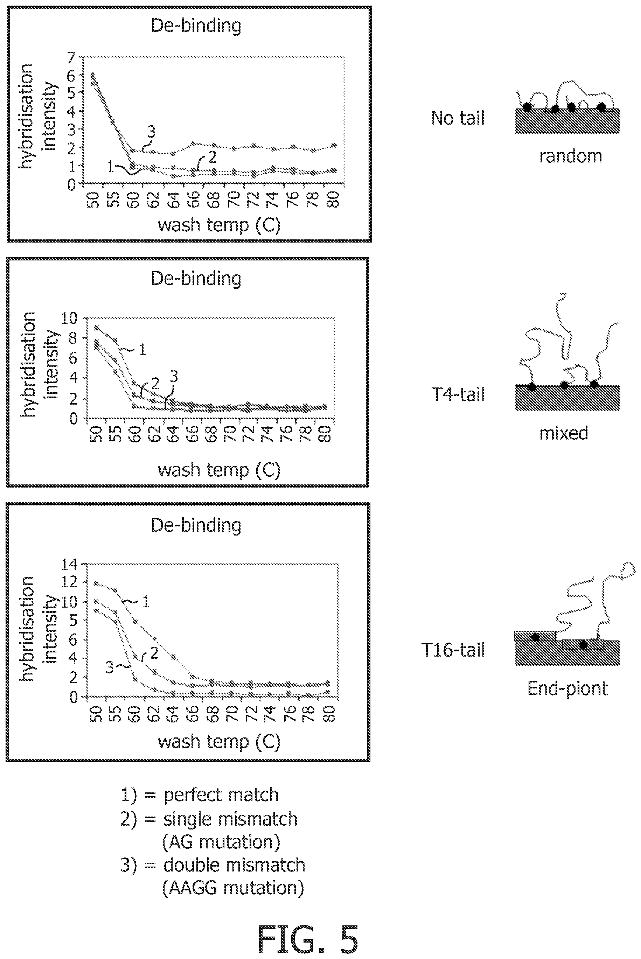

Fig. 5 depicts de-binding curves of complementary, single mismatch ((AG) mutation) and double mismatch ((AAGG) mutation) hybrids for capture probes with 0, 4 and 16 T's. The immobilization of the capture probes is schematically depicted along the graphs. The figure shows that increasing selectivity is obtained due to increased melting temperatures of the complementary probes as compared to mismatch probes.

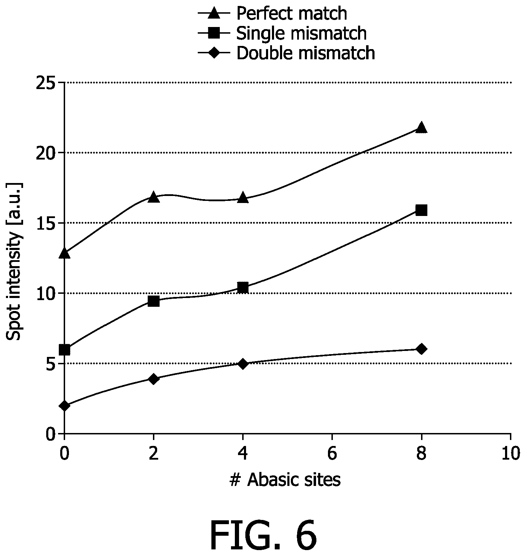

Fig. 6 shows the effect of the number of abasic sites (0, 2, 4, or 8) on the hybridization intensity of amplicons from a bacterial species to complementary as well as mismatch capture-probes.

### DETAILED DESCRIPTION OF THE INVENTION

The inventors have found that it is possible to determine the fate of spotted nucleic acids after the printing process is terminated and to evaluate the quality of the spotted probes.

Although the present invention will be described with respect to particular embodiments, this description is not to be construed in a limiting sense.

Before describing in detail exemplary embodiments of the present invention, definitions important for understanding the present invention are given.

As used in this specification and in the appended claims, the singular forms of "a" and "an" also include the respective plurals unless the context clearly dictates otherwise.

In the context of the present invention, the terms "approximately" denote an interval of accuracy that a person skilled in the art will understand to still ensure the technical effect of the feature in question. The term typically indicates a deviation from the indicated numerical value of ±20 %, preferably ±15 %, more preferably ±10 %, and even more preferably ±5 %.

It is to be understood that the term "comprising" is not limiting. For the purposes of the present invention the term "consisting of" is considered to be a preferred embodiment of the term "comprising of". If hereinafter a group is defined to comprise at least a certain number of embodiments, this is meant to also encompass a group which preferably consists of these embodiments only.

Furthermore, the terms "first", "second", "third" or "(a)", "(b)", "(c)", "(d)" etc. and the like in the description and in the claims, are used for distinguishing between similar elements and not necessarily for describing a sequential or chronological order. It is to be understood that the terms so used are interchangeable under appropriate circumstances and that the embodiments of the invention described herein are capable of operation in other sequences than described or illustrated herein.

In case the terms "first", "second", "third" or "(a)", "(b)", "(c)", "(d)" etc. relate to steps of a method or use there is no time or time interval coherence between the steps, i.e. the steps may be carried out simultaneously or there may be time intervals of seconds, minutes, hours, days, weeks, months or even years between such steps, unless otherwise indicated in the application as set forth herein below.

As has been set out above, the present invention concerns in one aspect a method for testing nucleic acids on a support which comprises (a) the immobilization of one or more nucleic acids on a solid support via crosslinking by heat or light or via chemical immobilization, wherein each of the immobilized nucleic acids includes a stretch of nucleotides of only one basetype, (b) the provision of a labeled oligonucleotide complementary to the stretch of nucleotides of only one basetype, wherein said labeled oligonucleotide is capable of forming a complex with each of the immobilized nucleic acids at the stretch of nucleotides of only one basetype, and (c) the determination of a value indicative for the interaction between said nucleic acid and the complementary oligonucleotide via the amount of labeled oligonucleotide being in complex with the immobilized nucleic acid.

The term "immobilizing a nucleic acid on a support" relates to the association of nucleic acid molecules to a supportive substrate via molecular interactions which position the nucleic acid at a specific area of the supportive substrate and concomitantly prevents a detaching of the nucleic acids, e.g. during washing, rinsing or chemical hybridization steps. Typically, such molecular interactions are based on covalent chemical bonds between structural elements or functional groups of the support material and the nucleic acid to be immobilized, e.g. corresponding functional groups of the nucleic acid, as known to the person skilled in the art.

The term "solid support" means that the support material is mainly of non-liquid consistence and thereby allows for an accurate and trackable positioning of the nucleic acid on the support material.

The term "crosslinking by heat or light" relates to an interaction between said support material and said nucleic acid via the formation of molecular interactions or bonds that link both structural elements together under the influence or driven by the energy provided by an energy source like heat or light.

Typically, crosslinking by heat is carried out via drying and subsequent baking of nucleic acid molecules on a substrate at certain temperatures. Drying and baking are believed to result in the nucleic acids becoming attached to the substrate by hydrophobic interaction, although the exact nature of the binding is not well understood. This procedure can be classified as a subform of physical adsorption. The term "physical adsorption" relates to a process involving initial separation and attraction steps, whereby the nucleic acid comes into proximity with the reactive groups, which are based on physical adsorptive processes. The adsorption of a biomolecule, e.g. a nucleic acid, onto a solid support may take place with practically any support material, since it has been observed that any such support material will interact with almost any surface. Typically, the level of interaction between support material and nucleic acid molecules varies depending on the nature and form of the support material and the size and chemical properties of the nucleic acids. The interaction is typically a five-stage procedure, comprising the steps of (i) transport of the molecule to the surface, (ii) adsorption to the surface, (iii) rearrangement of the adsorbed molecule, (iv) potential desorption of the adsorbed molecule and (v) transport of the desorbed molecule away from the surface.

Although the procedure implies, to a certain extent, that the potential for desorption is inherent, the binding is typically irreversible, depending on size of the molecule. The term "size of the molecule" within the context of adsorption interactions relates to the number of binding sites that are present. Although any one binding site may, in principle, dissociate from the surface of the substrate at any time, the effect of a large number of binding sites is that the molecule as a whole will remain bound. By applying energy in the form of heat, e.g. at a temperature of 40 to 150°C, preferably 50 to 120°C, more preferably 60 to 110°C, even more preferably 70 to 100°C and most preferably 80 to 90°C, the physical adsorption of the nucleic acid molecule to the support material may be enhanced and the time necessary for an efficient immobilization may be shortened. The crosslinking by heat may be carried out for any suitable period of time known to the person skilled in the art, e.g. 2 min to 12 hours, preferably 10 min to 8 hours, more preferably 30 min to 6 hours, even more preferably 45 min to 4 hours even more preferably 1 hour to 3 hours and most preferably for 2 hours. The crosslinking by heat or baking may be carried out by any suitable means known to the person skilled in the art, for example a drying chamber or an oven. In addition to the temperature, also other parameters like humidity, aeration or ventilation may be adjusted to suitable values known to the person skilled in the art. The crosslinking by heat or baking may also be combined with other forms of immobilization like crosslinking by light or chemical immobilization.

Crosslinking by light is performed by applying light of a typical wavelength, e.g. in a range of 150 to 550 nm, preferably in a range of 200 to 500 nm to nucleic acid molecules in order to induce an interaction between the molecules and support material. Typically, the induced interaction between the molecules and the support material is a covalent binding of the nucleic acid to the material. Crosslinking by light may, for example, be carried out by using UV light. UV crosslinking is one of the simplest ways to ensure covalent binding of a support material to a nucleic acid probe. Typically, the linkage proceeds through the bases of a nucleic acid molecule, e.g. thymine, guanine, adenine, cytosine or uracil residues, which react with corresponding and suitable functional chemical groups on the support material, as known to the person skilled in the art.

The presence and number of functional chemical groups on or inside the support material may be controlled and adjusted via suitable chemical activation processes. Such activation processes may, for instance, provide specifically localized functional groups on or within a support material and facilitate a specific interaction between the nucleic acids and the material within the context of these localized functional groups.

The presence and number of functional group on or inside the support material may also have an influence on the orientation and freedom of the immobilized nucleic acids. For example, the presence of a higher number of functional groups may lead to an immobilization at different points within the nucleic acid molecule. Furthermore, the presence of corresponding reactive elements within the nucleic acid molecule may be used for a control of the orientation of the nucleic acid molecule on the support material, e.g. an immobilization at the head or tail region or the 5' or 3' region of the nucleic acid molecule or an immobilization at the centre region alone or at the centre and the end regions at the same time.

A further parameter, which is of importance when crosslinking a nucleic acid molecule to a support material by light, is the amount of energy used for the irradiation. Typically, a person skilled in the art would be able to determine a suitable and optimal irradiation dose by following the indications provided by the manufacturers of irradiation equipment. For instance, the total dose to be applied onto the substrate may be calculated with the formula D = P · T , wherein D is the total dose applied onto the substrate in mJ/cm², P is the power of the light as applied on the support material in mW/cm² and T is the time during which the dose is applied in seconds. The power of the light as applied on the support material depends on the light source and the distance between the light source and the support material to be irradiated.

The light source may be any suitable light source known to the person skilled in the art, for instance, a mercury lamp, preferably a low-pressure mercury UV-lamp or a high-pressure mercury UV-lamp. The light source may also be a LED lamp, e.g. an UV-LED lamp.

The light source may emit a spectrum of wavelengths with predominant emission lines, e.g. at 254 nm or 365 nm. The light source may also be combined with a specific filter element in order to emit a specific emission line only. The filter may also be used to dampen the amount of energy to be applied onto the support material.

In addition to the wavelength and the amount of energy to be used, also other parameters like humidity, aeration or ventilation maybe adjusted to suitable values known to the person skilled in the art when carrying out a crosslinking by light. One key issue associated with the irradiation of support material is their moisture content. Since water absorbs light irradiation, in particular UV irradiation, a variation in the drying process may have influence on the outcome of the crosslinking process. The moisture content of a support material may be adjusted according to any suitable means known to the person skilled in the art. Preferably, crosslinking by light may be combined with a pre-drying procedure for a certain period of time in order to adjust the amount of water of liquid present.

The term "chemical immobilization" relates to an interaction between the support material and the nucleic acid based on chemical reactions. Such a chemical reaction does typically not rely on the input of energy via heat or light, but can be enhanced by either applying heat, e.g. a certain optimal temperature for a chemical reaction, or light of certain wavelength, as explained herein above. For example, a chemical immobilization may take place between functional groups on the support material and corresponding functional elements on the nucleic acid molecules. Such corresponding functional elements in the nucleic acid molecules may either be present in the molecule, e.g. as part of the chemical inventory of a nucleic acid molecule, or be additionally be introduced. An example of such a functional group is an amine group. Typically, the nucleic acid molecule comprises a functional amine group or is chemically modified in order to comprise a functional amine group. Means and methods for such a chemical modification are known to the person skilled in the art and can, for example, be derived from organic chemistry textbooks like Organische Chemie by Hart et al., 2007, Wiley-Vch or Organische Chemie by Vollhardt et al., 2005, Wiley-Vch.

The localization of said functional group within the molecule may be used in order to control and shape the binding behavior and/or orientation of the molecule, e.g. the functional group maybe placed at the end or tail region, at the 5' and/or 3' region of the molecule or in the centre of the molecule.

A typical reaction partner for a nucleic acid molecule comprises moieties which are capable of binding to nucleic acids, preferably to amine-functionalized nucleic acids. Examples of such support material are aldehyde, epoxy or NHS substrates. Such material is known to the person skilled in the art. Functional groups, which impart a connecting reaction between nucleic acid molecules which are chemically reactive by the introduction of an amine group, and a support material are known to the person skilled in the art.

An alternative reaction partner for a nucleic acid molecule may have to be chemically activated, e.g. by the activation of functional groups, available on the support material. The term "activated support material" relates to a material in which interacting or reactive chemical functional groups were established or enabled by chemical modification procedures as known to the person skilled in the art. For example, substrate comprising carboxylate groups has to be activated before use.

Furthermore, there are substrates available that contain functional groups that can react with specific moieties already present in the nucleic acids. Some of these reactions are enhanced by heat or UV. An example are amine groups on the surface of the substrate, which can be bound to specific bases in the DNA.

The above mentioned functional groups may also be localized or distributed differently, e.g. the support material may comprise amine groups and the nucleic acid molecules may be modified in order to encompass corresponding chemically reactive functional groups like expoxy, aldehyde or carboxylate groups.

The presence, number and localization of functional chemical groups on or inside the support material, which are capable of binding and immobilizing the nucleic acid molecules, maybe used in order to control and adjust the binding behavior of the nucleic acid molecules. The specific positioning of reactive chemical groups within the support material may be used in order to facilitate a specific interaction between the nucleic acids and the material within the context of these localized functional groups. Such positioning process may be used in order to provide an ordered array of specifically positioned nucleic acid molecules, e.g. via the use of liquid spotting equipment, preferably ink jet devices. Reactive chemical elements on or within the support material may also be masked by a blocking reagents and become available for chemical reaction with nucleic acid molecules after a deblocking or de-masking procedure. Alternatively, such chemical elements maybe activated by applying corresponding and suitable activation reagents known to the person skilled in the art.

The term "stretch of nucleotides of only one basetype" in the context of the present invention relates to portion of the nucleic acid molecule which is composed of only one base, e.g. thymine, guanine, adenine, cytosine or uracil or any chemical derivative thereof known to the person skilled in the art which is capable of interacting with a complementary base. Said portion of the nucleic acid molecule may have a variable length between only a few bases and more than 100 bases. The term "only one basetype" not only encompasses bases which are identical, but also bases or derivatives thereof which show a comparable chemical behavior in terms of interaction with complementary bases. The term thus relates in the exemplary case of thymines not only to the basetype or nucleotide thymine alone, but also to functionally equivalent derivates or modifications thereof. The term "functionally equivalent" relates to the capability of the base to establish a non-covalent connection with a complementary base, which is chemically similar to the non-covalent connection of the nucleotide or base it is derived from. Such functionally equivalent or modified bases may still be able to perform a hybridization binding with a complementary base.

The term "providing a labeled oligonucleotide" relates to the supply of an oligonucleotide comprising a chemical or physical element, which allows a distinction of the oligonucleotide from a background which does not comprise such an element. Such a distinction may preferably be based on optical differences, e.g. the emission of light from the label after stimulation or chemical activation or the emission of radioactive radiation. Preferably, such emitted light may be of a specific color, which is easily detectable from a contrasting background. The term "providing" also refers to the initiation and performance of an interaction procedure between such an oligonucleotide and one or more or preferably all nucleic acid molecules immobilized on a support material in accordance with the present invention. Particulars of such an interaction procedure are known to the person skilled in the art and can be derived from a molecular biology textbook like Sambrook et al., Molecular Cloning: A Laboratory Manual, 2001, Cold Spring Harbor Laboratory Press.

The term "oligonucleotide complementary to the stretch of nucleotides of only one basetpye" in the context of the present invention relates to a single stranded nucleic acid molecule of an intermediate number of residues, preferably of a length between 2 to 100 nucleotides, more preferably between 3 to 70 nucleotides, even more preferably of length of 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48 ,49, 50, 51, 52, 53, 54, 55, 56, 57, 58, 59, 60, 61, 62, 63, 64 or 65 nucleotides, most preferably of a length of 16 nucleotides, which offers a nucleotide sequence that is capable of binding via nucleic acid hybridization or non-covalent connection by hydrogen bonds to the immobilized nucleic acid as described herein above. The term "complementary" relates to the capability of the oligonucleotide to establish a non-covalent connection between the oligonucleotide and the immobilized nucleic acid via two or three hydrogen bonds depending on the bases present. Typically, adenine and thymine bases are non-covalently connected by two hydrogen bonds and guanine and cytosine bases are non-covalently connected by three hydrogen bonds. Uracil and adenine are typically non-covalently connected by two hydrogen bonds. Thus, if for example the stretch of nucleotides of only one basetype is composed of thymine nucleotides or functionally equivalent derivatives thereof, the labeled oligonucleotide maybe composed of adenine nucleotides or functionally equivalent derivatives thereof.

In the context of the present invention the term "complementary to the stretch of nucleotides of only one basetype" also encompasses oligonucleotides or portions of nucleic acid molecules which show one or more mismatches with respect to the chemical interaction rules of complementarity as defined herein above. The number of mismatches may vary depending on the length of the oligonucleotide and the size or length of the nucleic acid molecule. For example, an oligonucleotide may comprise within its stretch of complementary between 1% and 35% mismatch bases, preferably 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29% or 30% mismatch bases. The term "mismatch bases" relates to bases or nucleotides, which are not capable of establishing a non-covalently connection via hydrogen bonds to a base located in an opposition position on a second, interacting single stranded nucleic acid molecule. Such mismatch bases may be located throughout the oligonucleotide or on either of both termini of the oligonucleotide or in the center of the oligonucleotide. In accordance with the present invention such mismatch bases may have an influence on the overall interaction between the immobilized nucleic acid and the labeled oligonucleotide as defined herein above. Typically, said mismatch bases may reduce the strength of interaction of both molecules. The term "reduce" relates to a decrease in interaction of between 1 and 50%. The term, however, does not encompass a complete inhibition or obviation of interaction between the oligonucleotide in accordance with the present invention and the immobilized nucleic acid molecule.

The term "forming a complex with each of the immobilized nucleic acids at the stretch of nucleotides of only one basetype" in the context of the present invention relates to an interaction based on a non-covalent connection via hydrogen bonds between complementary bases within the immobilized nucleic acids and the oligonucleotide as defined herein above. Such a complex formation may be specific for all molecules which comprise regions of complementarity. The specificity depends on the chemical nature of the bases involved, the length of the molecules, the length and form of the complementary regions and parameters of the environment like temperature, pH, salt content and salt concentration or the present and concentration of further chemical compounds, as known to the person skilled in the art. The above mentioned parameters and factors maybe modified and adjusted in accordance with the present invention. Preferably, said parameters may be adjusted such that an oligonucleotide as defined herein above only binds to complementary stretches within a nucleic acid molecule if between 0% and 35% mismatch bases are present between both molecules, more preferably only if 0%, 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29% or 30% mismatch bases are present between both molecules.

The formation of a complex may accordingly also encompass regions of both molecules which are not complementary, as long as a detectable interaction between complementary regions of the molecules takes place. The term "each of the immobilized nucleic acids" relates to all immobilized nucleic acids possessing a stretch of nucleotides or bases of one basetype, which is complementary to the oligonucleotide sequence as defined herein above.

The term "determining a value indicative for the interaction between said nucleic acid and the complementary oligonucleotide" within the context of the present invention relates to the measurement of the degree of interaction between an immobilized nucleic acid and the complementary oligonucleotide as defined herein above. Such a degree of interaction allows drawing conclusions on several parameters. For example, the degree of interaction allows to check whether at a certain area or a specific spot of the support material a nucleic acid comprising a complementary sequence is present or not. Should the degree of interaction be extremely low or should there be no interaction at a specific area or a specific spot of the support material, such a result would be indicative for either the entire absence of a nucleic acid molecule or at least the absence of the region of complementarily within said molecule or the absence of functionality of the nucleic acid molecule.

Furthermore, the degree of interaction allows checking the quality of immobilized nucleic acids. Should the degree of interaction be low or at least not at its optimum, such a result would be indicative for a structurally impairment or modification of at least the region of complementarity present in said nucleic acid. Such a structural impairment in turn maybe indicative for an overall structural problem of the immobilized nucleic acid. A support material, biochip or microarray comprising such structurally impaired or modified nucleic acids as identified according to the present invention may, for example, be disregarded from further employment or scrutinized in a secondary testing approach according to the present invention, as described herein below.

The term "degree of interaction" means a numerical value which is derivable from the measurement of a physical, e.g. optical, or chemical signal within an area or zone of the substrate in which a nucleic acid has been immobilized after a labeled oligonucleotide according to the invention was provided as defined herein above, i.e. after the oligonucleotide was brought into the proximity of immobilized nucleic acid molecules in order to allow the formation of a complex, in comparison to the measurement of such a signal in an area in which no nucleic acid was immobilized, i.e. a background area. The presence of labeled elements within the oligonucleotide may accordingly be measured and detected with corresponding, suitable methods as known to the person skilled in the art. Accordingly, only in those areas in which an interaction between the labeled oligonucleotide and the immobilized nucleic acid takes place, such a physical or chemical signal can be detected. The numerical value to be measured depends on the label element which is used on the oligonucleotide, i.e. the absolute strength of the signal. Said value may also refer to the signal strength which is adjusted to the background signal at one or more areas of the substrate, preferably at several distinct position of the support material which do not comprise immobilized nucleic acids.

Furthermore, said value may be adjusted to a control value obtained in an interaction reaction in which the labeled oligonucleotide optimally interacts with the immobilized nucleic acid, e.g. in which all bases of the oligonucleotide are connected or hybridizing with an immobilized nucleic acid molecule and/or in which essentially no de-binding of the oligonucleotide takes place.

A degree of interaction at a certain, specific area of the support material of 0% to 5%, preferably of 0% to 3% calculated with respect to a control value as defined herein above can be seen as indicative for the entire absence of a nucleic acid at said certain area or spot of the support material or the absence of the region of complementarity within said nucleic acid. A degree of interaction of 5% to 80%, preferably of 10% to 70% may be seen as indicative for the presence of a nucleic acid molecule at a specific area or spot of the support material, which is structurally impaired at least in the region of complementarity present in said nucleic acid or which comprises only a subportion of said region. Such a degree of interaction is further indicative for problems which occurred during the depositioning or immobilization process. A nucleic acid which shows an intermediate degree of interaction of 5% to 80%, preferably of 10% to 70% in accordance with the present invention may still be able to optimally interact with a second oligonucleotide which is complementary to a specific stretch of nucleotides outside the stretch of nucleotides of only one basetype, since the degree of interaction as defined herein above is only calculated within the context of the stretch of nucleotides of only one basetype. Nucleic acids showing such an intermediate degree of interaction may additionally be tested with a secondary, specifically binding oligonucleotide, e.g. an oligonucleotide which is complementary to a specific stretch of nucleotides outside the stretch of nucleotides of only one basetype, in order to figure out whether the specific stretch of nucleotides is capable of binding with a higher degree of interaction, i.e. whether said intermediate degree of interaction is due to constraints within the stretch of nucleotides of only one basetype or due to a structural impairment at such a second site as well. Such a secondary interaction testing may also be carried out in cases in which only a very low or no degree of interaction could be measured with an oligonucleotide complementary to the stretch of nucleotides of only one basetype. Details of a corresponding secondary interaction are described herein below.

A degree of interaction at a certain, specific area of the support material of 80% to 100%, preferably of 90% to 100% calculated with respect to a control value as defined herein above can be seen as indicative for the presence of a nucleic acid molecule at said specific area or spot of the support material, which is structurally not impaired at least in the region of complementarity present in said nucleic acid. In certain cases the degree of interaction may also be above 100%, e.g. between 100% and 150%. Such a result may be obtained for instance if the control value is derived from a signal which is less strong than the strongest signal in the detection area or if the control value is derived from an averaged or normalized signal whose average signal value is lower than the strongest signal in the detection area.

The term "signal" means any chemically or physically, preferably optically, distinguishable difference between two points or areas on the surface of the support material or within the support material. The measurement or detection of any such signal can be performed with any suitable means known to the person skilled in the art. For example a signal may be detected with a microarray scanner apparatus or CCD optical equipment. In order to analyze and compare the detected signal appropriate computer equipment and programs may be used according to the necessities. Such computer equipment and programs are known to the person skilled in the art.

The term "interaction between said nucleic acid and the complementary oligonucleotide" relates to the presence or absence of immobilized nucleic acids and also to the quality of the immobilized nucleic acids in terms of structural constraints and impairments within the region of complementarity. The method for testing nucleic acids of the present invention as defined herein above accordingly allows both, an "all or nothing"-discrimination between existing and non-existing deposition and immobilization pattern on a substrate and a "quality control"- discrimination between several degrees of interaction between the immobilized nucleic acid molecules and the labeled oligonucleotide as defined herein above. Low or moderate degrees of interaction may be seen as indicative for a lower quality of the immobilized nucleic acids, which may lead to subsequent problems during interaction procedures with specifically binding oligonucleotides.

Furthermore, steps (a), (b) and (c) of the method for testing nucleic acids of the present invention as described herein above may be carried out without any time- or time interval-coherence between the steps, i.e. all steps or certain subgroups of steps maybe carried out simultaneously or there may be any suitable time interval between steps (a) and/or (b) and/or (c). For instance, step (b) may be performed after a time interval of seconds, minutes, hours, days, weeks, months or even years after the performance of step (a). The same applies to step (c) with respect to step (a) and step (b).

In a specific embodiment, step (a) is carried out first, followed by step (b) and step (c).

Preferably, subsequent steps (b) to (c) are carried out after one hour to 12 months after step (a) has been initiated or terminated.

In a further aspect the present invention relates to a kit for testing nucleic acids on a support. Said kit comprises an array of nucleic acids immobilized on a solid support via crosslinking by heat or light or via chemical immobilization as defined herein above, wherein each of the immobilized nucleic acids includes a stretch of nucleotides of only one basetype as defined herein above and a labeled oligonucleotide complementary to the stretch of nucleotides of only one basetype, wherein said labeled oligonucleotide is capable of forming a complex with each of the immobilized nucleic acids at the stretch of nucleotides of only one basetype. The kit may additionally comprise ingredients and components necessary for a testing reaction as defined herein above, e.g. buffers like hybridization buffers, washing liquids and/or components capable of detecting label elements, or a package information leaflet comprising information on the employment of the kit. The kit may be provided in any suitable form known to the person skilled in the art. For example, the kit may be provided as an open or closed cartridge. A closed cartridge kit may comprise several compartments in which one or more of the above indicated ingredients may be stored.

In another aspect the present invention relates to the use of a labeled oligonucleotide as defined herein above which is complementary to a stretch of nucleotides of only one basetype as defined herein above for testing the interaction between the complementary oligonucleotide and nucleic acids immobilized on a solid support via crosslinking by heat or light or via chemical immobilization as defined herein above, wherein each of the immobilized nucleic acids includes a stretch of nucleotides of only one basetype and wherein said labeled oligonucleotide is capable of forming a complex with each of the immobilized nucleic acids at said stretch of nucleotides of only one basetype.

The use of a labeled oligonucleotide which is complementary to a stretch of nucleotides of only one basetype for testing the interaction between the complementary oligonucleotide and nucleic acids immobilized on a solid support via crosslinking may comprise the determination of a value indicative for the amount of labeled oligonucleotide being in complex with an immobilized nucleic acid as defined herein above.

The nucleic acid in accordance with a preferred embodiment of the present invention may be a single stranded DNA, RNA, PNA, CNA, HNA, LNA or ANA. The DNA may be in the form of, e.g. A-DNA, B-DNA or Z-DNA. The RNA may be in the form of, e.g. p-RNA, i.e. pyranosysl-RNA or structurally modified forms like hairpin RNA or a stem-loop RNA.

The term "PNA" relates to a peptide nucleic acid, i.e. an artificially synthesized polymer similar to DNA or RNA which is used in biological research and medical treatments, but which is not known to occur naturally. The PNA backbone is typically composed of repeating N-(2-aminoethyl)-glycine units linked by peptide bonds. The various purine and pyrimidine bases are linked to the backbone by methylene carbonyl bonds. PNAs are generally depicted like peptides, with the N-terminus at the first (left) position and the C-terminus at the right.

The term "CNA" relates to an aminocyclohexylethane acid nucleic acid. Furthermore, the term relates to a cyclopentane nucleic acid, i.e. a nucleic acid molecule comprising for example 2'-deoxycarbaguanosine.

The term "HNA" relates to hexitol nucleic acids, i.e. DNA analogues which are built up from standard nucleobases and a phosphorylated 1,5-anhydrohexitol backbone.

The term "LNA" relates to locked nucleic acids. Typically, a locked nucleic acid is a modified and thus inaccessible RNA nucleotide. The ribose moiety of an LNA nucleotide may be modified with an extra bridge connecting the 2' and 4' carbons. Such a bridge locks the ribose in a 3'-endo structural conformation. The locked ribose conformation enhances base stacking and backbone pre-organization. This may significantly increase the thermal stability, i.e. melting temperature of the oligonucleotide.

The term "ANA" relates to arabinoic nucleic acids or derivatives thereof. A preferred ANA derivative in the context of the present invention is a 2'-deoxy-2'-fluoro-beta-D-arabinonucleoside (2'F-ANA).

In a further preferred embodiment nucleic acid molecules may comprise a combination of any one of single stranded DNA, RNA, PNA, CNA, HNA, LNA and ANA. Particularly preferred are mixtures of LNA nucleotides with DNA or RNA bases.

In a further preferred embodiment the nucleic acid molecules as defined herein above may be in the form of short oligonucleotides, long oligonucleotides or polynucleotides.

The stretch of nucleotides of one basetype in accordance with a preferred embodiment of the present invention maybe composed of thymine, uracil, guanine, adenine or cytosine bases only. The stretch of nucleotides of one basetype may additionally also be composed of functional equivalents of thymine, uracil, guanine, adenine or cytosine bases as defined herein above or a combination of thymine and its functional equivalents, uracil and its functional equivalents, guanine and its functional equivalents, adenine and its functional equivalents or cytosine and its functional equivalents. The term "functional equivalent" relates to a base which is capable of establishing a non-covalent connection with a complementary base that is chemically similar to the non-covalent connection of the nucleotide or base it is derived from.

In a particularly preferred embodiment the stretch of nucleotides of only one basetype is a stretch of thymines, uracils or guanines or combinations thereof with its respective functional equivalents. Even more preferred is a stretch of nucleotides of only one basetype, composed of thymines or combinations or thymines with its functional equivalents.

Nucleic acids of only one basetype in accordance with a further preferred embodiment of the invention may have a length up to 100 nucleotides, particularly preferably up to 50 nucleotides and even more preferably from 10 to 20 nucleotides. Also preferred is a length of 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39 or 40 nucleotides. Most preferred is a length of 16 nucleotides.

Crosslinking used for the immobilization of one or more nucleic acids on a solid support in accordance with another preferred embodiment of the present invention may be a crosslinking by light performed at a wavelength in a range of 200-300 nm or a subrange thereof, e.g. a subrange of 200 to 220 nm, 220 to 240 nm, 240 to 260 nm, 260 to 280 nm, 280 to 300 nm. Such a crosslinking maybe considered as a classical UV-crosslinking. The wavelength of the light to be used may be determined primarily by the choice of lamps. For instance, in order to establish a wavelength in the spectrum of 200-300 nm a low-pressure mercury UV-lamp maybe used. Such a lamp typically emits not only one wavelength, but a spectrum of wavelengths, as the person skilled in the art would know. The term "spectrum of 200-300 nm" relates to such a typical spectrum emitted from a low-pressure mercury UV-lamp. Alternatively, the light may also be emitted from a UV LED, which may have a different emission spectrum or any other lamp or light source known to the person skilled in the art as long the majority of the emitted wavelengths are within the range of 200-300 nm. In a particularly preferred embodiment the prominent emission line is at 254 nm within said emission spectrum of 200-300 nm. Further preferred is a crosslinking approach in which a light source is used which does not emit an entire spectrum of wavelengths, but only specific wavelengths, particularly preferably a wavelength of 254 nm. Such a confinement may be achieved by using specific lamp or LED models or by employing filter elements which allow solely the passage of defined wavelengths, as the person skilled in the art would know.

Crosslinking of nucleic acids at a wavelength of 200 - 300 nm, in particular at 254 nm may preferably be used in order to immobilize nucleic acid molecules comprising uracil, thymine, guanine, cytosine or adenine bases, more preferably nucleic acids comprising a stretch of only one base type composed of uracil, thymine, guanine, cytosine or adenine bases. In a more preferred embodiment, crosslinking of nucleic acids at a wavelength of 200 - 300 nm, in particular at 254 nm may preferably be used in order to immobilize nucleic acid molecules comprising a stretch of only one base type composed of uracil, thymine or guanine bases, even more preferably nucleic acids comprising a stretch of only one base type composed of uracil or thymine bases. Most preferred are nucleic acid molecules comprising a stretche of only one base type composed of uracil bases, since it has been found that a stretch of only one base type composed of uracil may be immobilized more efficiently to a support material at a wavelength of 254 nm than a stretch of only one base type composed of thymine, which in turn may be immobilized more efficiently to a support material at said wavelength than an a stretch of only one base type composed of guanine, cytosine or adenine.

Crosslinking used for the immobilization of one or more nucleic acids on a solid support in accordance with another preferred embodiment of the present invention may be a crosslinking by light performed at a wavelength of 300-500 nm or a subrange thereof, e.g. a subrange of 300 to 320 nm, 320 to 340 nm, 340 to 360 nm, 360 to 380nm, 380 to 400 nm, 400 to 420 nm, 420 to 440 nm, 440 to 460 nm, 460 to 480 nm, 480 to 500 nm. Such a crosslinking may be considered as a non-classical UV or long wavelength-crosslinking. The wavelength of the light to be used may be determined primarily by the choice of lamps. For instance, in order to establish a wavelength in the spectrum of 300-500nm a high-pressure mercury UV-lamp may be used. Such a lamp typically emits not only one wavelength, but a spectrum of wavelengths, as the person skilled in the art would know. The term "spectrum of 300-500 nm" relates to such a typical spectrum emitted from a high-pressure mercury UV-lamp. Alternatively, the light may also be emitted from a LED, which may have a different emission spectrum or from any other lamp or light source known to the person skilled in the art as long the majority of the emitted wavelengths are within the range of 300-500 nm. In a particularly preferred embodiment the prominent emission line is at 365 nm within said emission spectrum of 300-500 nm. Further preferred is a crosslinking approach in which a light source is used which does not emit an entire spectrum of wavelengths, but only specific wavelengths, particularly preferably a wavelength of 365 nm. Such a confinement may be achieved by using specific lamp or LED models or by employing filter elements which allow solely the passage of defined wavelengths, as the person skilled in the art would know.

Crosslinking of nucleic acids at a wavelength of 300 - 500 nm, in particular at 365 nm may preferably be used in order to immobilize nucleic acid molecules comprising uracil, thymine, guanine, cytosine or adenine nucleotides, more preferably nucleic acids comprising a stretch of only one base type composed of uracil, thymine, guanine, cytosine or adenine nucleotides. In a more preferred embodiment, crosslinking of nucleic acids at a wavelength of 300 - 500 nm, in particular at 365 nm may preferably be used in order to immobilize nucleic acid molecules comprising a stretch of only one base type composed of guanine, uracil or thymine bases, even more preferably nucleic acids comprising a stretch of only one base type composed of guanine or uracil bases. Most preferred are nucleic acid molecules comprising a stretch of only one base type composed of guanine bases, since it has been found by the present inventors that a stretch of only one base type composed of guanine may be immobilized more efficiently to a support material at a wavelength of 365 nm than an a stretch of only one base type composed of uracil, which in turn may be immobilized more efficiently to a support material at said wavelength than a stretch of only one base type composed of thymine, which in turn may be immobilized more efficiently to a support material at said wavelength than an a stretch of only one base type composed of cytosine or adenine.

Crosslinking used for the immobilization of one or more nucleic acids on a solid support in accordance with another preferred embodiment of the present invention may be a crosslinking by light performed at a wavelength of 200-300 nm carried out by using an amount of energy ranging from 0.05 to 1.5 Joule/cm², more preferably from 0.075 to 1.0 Joule/cm² even more preferably from 0.1 to 0.6 Joule/cm² and most preferably at 0.3 Joule/cm². In a particularly preferred embodiment crosslinking is carried out at a wavelength of 254 nm by using an amount of energy of 0.3 Joule/ cm².

Crosslinking used for the immobilization of one or more nucleic acids on a solid support in accordance with another preferred embodiment of the present invention may be a crosslinking by light performed at a wavelength of 300-500 nm carried out by using an amount of energy ranging from 0.5 to 15 Joule/cm², more preferably from 2.0 to 12 Joule/cm² even more preferably from 4 to 10 Joule/cm² and most preferably at 5 Joule/cm². In a particularly preferred embodiment crosslinking is carried out at a wavelength of 365 nm by using an amount of energy of 5 Joule/ cm².

As stated herein above, the power of the light as applied on the support material and thus the amount of energy applied depends, inter alia, on the distance between the light source and the support material to be irradiated. The distance between the used light source and the support material may be suitably adjusted according to parameters known to the person skilled in the art. Preferably a distance between 5 cm and 1 m is used, more preferably between 10 cm and 500 cm, even more preferably a distance between 20 cm and 200 cm. Further preferred is a distance between 10 cm and 150 cm. Most preferred is a distance of 50 cm.

The chemical immobilization of a nucleic acid molecule to the support material may in accordance with a further preferred embodiment of the invention be carried out by a coupling between an amine-modified nucleic acid and an element of the support material comprising a corresponding functionality, i.e. a functional chemical group which predominantly interacts with amine-modified nucleic acid molecules. The term "amine modified" relates to the introduction, activation or modification of amine groups within the nucleic acid molecule with the purpose of establishing reactive functional amine groups. Such amine groups may, for example, be introduced throughout the length of the molecule. Preferably the groups are introduced at both or one of the termini of the molecule or at its center. Such a modification may be used in order to control and shape the binding behavior of the molecule on the support A suitable functional chemical group which predominantly interacts with amine-modified nucleic acid would be known to the person skilled in the art and can be derived from organic chemistry textbooks, e.g. from Organische Chemie by Hart et al., 2007, Wiley-Vch or Organische Chemie by Vollhardt et al., 2005, Wiley-Vch In a particularly preferred embodiment the immobilization of a nucleic acid molecule which is amine-modified onto a support material is performed via an interaction of said amine groups on the nucleic acid molecule and epoxy, aldehyde, carboxylate or NHS groups on the support material. The term "NHS" relates to N-hydroxysuccinimide, which is a compound used as an activating reagent for carboxylic acids. Activated acids (basically esters with a good leaving group) can react with amines to form amides for example, whereas a normal carboxylic acid would just form a salt with an amine. Typically, an NHS-activated acid is synthesized by mixing NHS with a desired carboxylic acid and a small amount of an organic base in an anhydrous solvent. A dehydrating agent such as dicyclohexylcarbodiimide (DCC) or ethyl(dimethylaminopropyl) carbodiimide (EDC) may subsequently be added to form a highly unstable activated acid intermediate. NHS reacts to form a less labile activated acid. Such an ester with an acid and NHS, i.e. a succinate ester, is stable enough to be purified and stored at low temperatures in the absence of water and, as such is suitable for the fixation of nucleic acids on a support material which afterwards may be subjected to washing and/or hybridization procedures.

The nucleic acid to be immobilized on the support material may according to a further preferred embodiment be represented by the formula I:

5'-Yₙ-Xₘ-Bᵣ-Xₚ-Z_{q}-3'

In formula I Y and Z are stretches of nucleotides of only one basetype, wherein Y and Z can be of the same or of a different basetype; X is a spacer; B is a sequence of more than one basetype and n, m, r, p and q are numbers of nucleotides in the nucleic acid, for which the following conditions may apply: n, m, p, q, r >1; n, m, r > 1 and p, q = 0; p, q, r >1 and n, m = 0; n, q, r > 1 and m, p = 0; n, r > 1 and m, p, q = 0; q, r > 1 and n, m, p = 0. The term "stretch of nucleotides of only one basetype" has already been defined herein above and relates to nucleotides composed of only one kind of base, e.g. thymine, guanine, adenine, cytosine or uracil or any functional equivalent derivative thereof.

Preferably, the stretches Y and/or Z may be used for immobilization of the nucleic acid due to the presence of nucleotides of only one basetype. More preferably, the stretches Y and/or Z may be composed of uracil or thymine bases, even more preferably of uracil if the immobilization is to be carried out by crosslinking at a wavelength of 200-300nm, e.g. at 254 nm or they maybe composed of guanine or uracil, more preferably of guanine if the immobilization is to be carried out by crosslinking at a wavelength of 300-500nm, e.g. at 365 nm.

Y and Z may be present at the same time on the same nucleic acid molecule. Such a format maybe used for a simultaneous crosslinking via the stretches of only one basetype at both termini of the molecule. In a further preferred embodiment Y and Z may be composed of different basetypes, i.e. Y maybe, for example, of basetype uracil, whereas Z may be of basetype guanine or vice versa. Such a nucleic acid may, for example, be immobilized at different wavelengths, preferably at 254 nm and 365 nm, and accordingly lead to a distinguishable orientation of the nucleic acid. Such nucleic acids may also be used for testing of influences of the nucleic acid orientation and immobilization approach on the capability of forming complexes with a complementary oligonucleotide in accordance with the present invention.

In a preferred embodiment Y and Z may be identical in length or may be different in length. Y and/or Z may have a length of up to 100 nucleotides, more preferably of up to 50 nucleotides, even more preferably of 8 to 30 nucleotides. Also preferred is a length of 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39 or 40 nucleotides. Most preferred is a length of 16 nucleotides.

In a format which comprises elements Y and Z at both termini the nucleic acid molecule comprises in its center a region of specific nucleotides B as depicted herein above in formula I. Alternatively, region B may be connected to only one of Y or Z and thus be located at the terminus of the molecule. The region B maybe used for specific detection reactions in a classical hybridization or microarray approach, i.e. for interaction reactions with oligonucleotides which specifically bind to their complementary region residing within element B. The length and chemical nature of Y and/or Z may have an influence on the flexibility of zone B and, hence, may be used in order to optimize the specific interaction within this zone, e.g. the specific hybridization reactions using complementary oligonucleotides. In a preferred embodiment B has a length of 4 to 90 nucleotides, more preferably a length of 4 to 50 nucleotides, even more preferably of 20 to 30 nucleotides. Preferred lengths are also 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39 or 40 nucleotides. Most preferred is a length of 25 nucleotides.

Thus, the stretch of nucleotides of only one basetype as defined herein above may be located in accordance with a particular embodiment of the present invention at either of both termini of the nucleic acid molecule, i.e. at either the 3' or the 5' of the immobilized nucleic acid. More preferably the stretch of nucleotides of only one basetype may be located at the 5' end of the nucleic acid molecule.

Element(s) X of Formula I of the present invention may additionally be present as spacer element(s), i.e. as regions comprising sequences of undefined nature. More preferably element X may be composed of abasic nucleotides. The term "abasic" relates to positions in the nucleic acid molecule, at which no basic residue is present. Abasic regions or stretches of a nucleic acid are, thus, only composed of sugar phosphate backbone elements. Such an abasic structure may have a positive influence on the flexibility of the entire molecule, in particular with respect to element B of the molecule. The inventors could show that the presence of abasic sites have a positive influence on the capability of the immobilized molecule to specifically interact with or hybridize to a target probe (see Example 5 and Fig. 6). A separation of the portions of the molecule used for immobilization, e.g. Y or Z of formula I, form the portion(s) of the molecule used for specific hybridization, e.g. B of formula I, by way of introducing spacer elements comprising abasic sites may significantly decrease unspecific hybridization reactions in the portion of the molecule used for specific hybridization, e.g. B of formula I.

Spacer elements Xm and Xp may entirely be composed of abasic sites or partially be composed of abasic sites. Is the spacer element partially composed of abasic sites the basic portions of the spacer element may be composed of nucleotides of only one basetype or maybe composed of nucleotides of different basetypes. Abasic sites as defined herein above may either be accumulated in one stretch or be dispersed within a spacer element or, alternatively, also be present throughout the entire molecule as depicted in formula I. Preferably, the abasic sites are located within the spacer elements X and are accumulated in 1 or 2 stretches.

Preferably, the number of abasic sites within a molecule as depicted in formula I may be between 1 and 30, more preferably between 1 and 20, even more preferably such a molecule may comprise 1, 2, 3, 4, 5, 6, 7, 8, 9, 10, 11, 12, 13, 14, 15, 16, 17, 18, 19 or 20 abasic sites.

Spacer elements Xm and Xp maybe identical in chemical nature and length or may be different in chemical nature and length. Preferably, spacer elements Xm and Xp are of an equal length of 1 to 50 nucleotides, more preferably of a length of 1 ,2 ,3 ,4, 5, 6, 7, 8, 9, 10, 11, 12, 13, 14, 15, 16, 17, 18, 19, 20, 21, 22, 23, 24, 25, 26, 27, 28, 29 or 30 nucleotides. In a further embodiment, in case q=0, i.e. no sequence element Z as depicted in formula I is present, also a terminal spacer is avoided, i.e. p=0. Similarly, in case n=0, i.e. no sequence element Y as depicted in formula I is present, also a terminal spacer is avoided, i.e. m=0.

The nucleic acid to be immobilized and/or the oligonucleotide complementary thereto may according to a further preferred embodiment of the invention comprise one or more labels at either or both of the termini, preferably at the 5' terminus. Alternatively, said nucleic acid molecules or oligonucleotides may also comprise one or more labels at any position throughout the molecule. Preferably said nucleic acid molecule or oligonucleotide comprises between 1 and 10 labels, which may either be identical or different or any combination thereof. More preferably, the nucleic acid molecule or oligonucleotide comprises between 1 and 5 labels, even more preferably 2 labels and most preferably only one label.

Said labels maybe radioactive, fluorescent or chemiluminescent labels. The term "radioactive label" relates to labels emitting radioactive radiation, preferably composed of radioactive isotopes. The term "radioactive isotope" in the context of the label relates to any such factor known to the person skilled in the art. More preferably, the term relates to N-15, C-13, P-31 or I-131.

The term "fluorescent label" relates to chemically reactive derivatives of a fluorophores. Typically common reactive groups include amine reactive isothiocyanate derivatives such as FITC and TRITC (derivatives of fluorescein and rhodamine), amine reactive succinimidyl esters such as NHS-fluorescein, and sulfhydryl reactive maleimide activated fluors such as fluorescein-5-maleimide. Reaction of any of these reactive dyes with another molecule results in a stable covalent bond formed between a fluorophore and a labeled molecule. Following a fluorescent labeling reaction, it is often necessary to remove any nonreacted fluorophore from the labeled target molecule. This may be accomplished by size exclusion chromatography, taking advantage of the size difference between fluorophore and labeled nucleic acid or oligonucleotide. Fluorophores may interact with the separation matrix and reduce the efficiency of separation. For this reason, specialized dye removal columns that account for the hydrophobic properties of fluorescent dyes may be used. A particular advantage of fluorescent labels is that signals from fluorescent labels do not disperse. The lack of dispersal in the fluorescent signal permits, for example, a denser spacing of probes on a support. Another advantage of fluorescent probes is that an easy multiple-color hybridization detection may be carried out, which permits direct quantitative determination of the relative abundance of oligonucleotides forming a complex with the nucleic acid molecules immobilized on the support material. In a particularly preferred embodiment the fluorescent labels FITC, Fluorescein, Fluorescein-5-EX, 5-SFX, Rhodamine Green-X, BodipyFL-X, Cy2, Cy2-OSu, Fluor X, 5 (6) TAMRA-X, Bodipy TMR-X, Rhodamine, Rhodamine Red-X, Texas Red, Texas Red-X, Bodipy TR-X, , Cy3-OSu, Cy3.5-OSu, Cy5, Cy5-Osu, Alexa fluors, Dylight fluors and/or Cy5.5-OSu maybe used. These labels may be used either individually or in groups in any combination.

The term "chemiluminescent label" relates to a label which is capable of emitting light (luminescence) with a limited emission of heat as the result of a chemical reaction. Preferably, the term relates to luminol, cyalume, oxalyl chloride, TMAE (tetrakis (dimethylamino) ethylene), pyragallol, lucigenin, acridinumester or dioxetane.

In a particularly preferred embodiment both entities, the nucleic acid molecule and the oligonucleotide as defined herein above, may each be labeled with a different label, typically with two different labels that are optically or chemically distinguishable. Such distinguishable labels may be present at different locations within the nucleic acid molecule and the oligonucleotide. Thus, if the nucleotide is, for example, labeled with Cy2, the oligonucleotide may be labeled with Cy5. These labels may be used, for instance, for the detection of molecular processes during the interaction between the immobilized nucleic acid molecule and the complementary, binding oligonucleotide.

Such differential labeling further provides the opportunity to co-localize both, the immobilized nucleic acid and any binding oligonucletide. Such an approach may also be used in order to obtain values for the degree of interaction as defined herein above.

In a further preferred embodiment a control nucleic acid may be labeled with a certain label, preferably a fluorescent label, and the test oligonucleotide in accordance with the present invention maybe labeled with a different, optically distinguishable label or the same label. If the signal obtained from the control nucleic acid is taken as 100%, any signal obtained from an interaction between an immobilized nucleic acid and said labeled oligonucletide maybe normalized against said value in order to define an alternative value for the degree of interaction. Additionally, a background signal derived from areas of the support material where no nucleic acids are immobilized maybe subtracted.

The support material in accordance with another preferred embodiment of the present invention may be a solid material or a substrate comprising functional chemical groups, preferably amine groups or amine-functionalized groups. The term "amine-functionalized group" relates to groups which have been functionalized with amines, i.e. which have adopted by chemical modification the function of amines. These amines or amine groups may be primary or secondary amines. Furthermore, the support material or substrate may comprise photoactivatable compounds which may be used for an interaction between the support material and the nucleic acid molecules. Suitable photoactive chemicals, as known to the person skilled in the art, can be used as connector molecules. Examples of such molecules are photobiotin, or reactive moieties like succinimidyl-6-[4'-azido-2'-nitrophenylamino] hexanoate into the support material.

Photobiotin is composed of a biotinyl group, a linker group, and a nitrophenyl azide group which is photoactivatable. It is generally used for patterning molecules onto solid substrates. Typically, UV lasers stimulate photobiotin to attach various surfaces. The attachment procedure normally takes place in aqueous solutions. Photobiotin is a biotin species, which is photoactivatable and can be used to biotinylate nucleic acids and molecules, in particular those which do not have amine or sulfhydryl groups present to engage in coupling. When exposed to strong light, biotin's aryl azide groups are converted to an aryl nitrene, which is extremely reactive. This process can be used to label a molecule with biotin, e.g. nucleic acid molecules.

These above mentioned compounds may react with the nucleic acid molecules and immobilize the molecules on the substrate.

In a further preferred embodiment, the support material comprises psoralen. Psoralen is a bifunctional photochemical crosslinking reagent for nucleic acids. It intercalates within nucleic acid helices, and upon irradiation with long-wavelength (365 nm) UV-light forms covalent bonds to pyrimidine bases. Preferably, psoralen may be used in order to immobilize nucleic acids via crosslinking by light at a wavelength of 300-500 nm, more preferably at a wavelength of 365 nm.

A preferred support material is a porous support material or porous substrate. Particularly preferred is nylon, e.g. Nytran N® or Nytran SPC® or Biodyne C®. A further preferred support material or substrate type is a non-porous substrate. Particularly preferred among non-porous substrates are glass, poly-L-lysine coated material, nitrocellulose, polystyrene, cyclic olefin copolymers (COCs), cyclic olefin polymers (COPs), polypropylene, polyethylene and polycarbonate.

Nitrocellulose membranes are the traditional membranes which are generally used fort transfer techniques like Southern blotting. Methods to achieve nucleic acid binding to nitrocellulose, usually by means of physical adsorption, are widely known form the prior art. The principal advantages of nitrocellulose are its ready availability and familiarity. The use of nitrocellulose membranes with radioactive methods of signal detection is well established.

As an alternative to nitrocellulose membranes nylon may be used as a substrate for nucleic acid binding owing to its greater physical strength and binding capacity, and the wider range of available surface chemistries offered, which optimizes nucleic acid attachment. Immobilization on nylon membranes can be performed, for example, via crosslinking by light, in particular UV-crosslinking, or chemical activation. Immobilization on nylon has been demonstrated to be very durable during repeated probe stripping.

The means by which macromolecules bind to bulk material like, for instance, polystyrene is not well understood. An allocation of binding capacity for bulk materials or its enhancement may be achieved by the provision of functional groups, preferably amine groups, which are made available, e.g. by a coating process or surface treatment or spraying etc. A preferably used coating material is poly-L-lysine, which belongs to the group of cationic surfactants. It contains positively charged hydrophilic (amino) groups and hydrophobic (methylene) groups and is known to interact with nucleic acid molecules.

As bulk material any suitable material known to the person skilled in the art may be used. Typically, glass or polycarbonate or cyclic olefin copolymer or cyclic olefin polymer or polystyrene is used. Polystyrene is a hydrophobic material suitable for binding negatively charged macromolecules because it normally contains few hydrophilic groups.

For nucleic acids immobilized on glass slides, it is furthermore known that by increasing the hydrophobicity of the glass surface the DNA immobilization may be increased. Such an enhancement may permit a relatively more densely packed formation.

In addition to a coating or surface treatment with poly-L-lysine, bulk material, in particular glass, may be treated by silanation, e.g. with epoxy-silane or amino-silane or by silynation or by a treatment with polyacrylamide.

In a further specific embodiment of the present invention bulk material may also be covered with or coated with membrane material as mentioned herein above.

In a further preferred embodiment the formation of a complex in accordance with the present invention is a hybridization method. Hybridization reactions typically rely, inter alia, on the nature and concentration of hybridization buffers and on the hybridization temperature.

A hybridization buffer to be used in the context of the present invention, e.g. in the context of the method for testing or the method for analyzing or comprised in a kit of the present invention typically comprises salts, which are able to enhance the hybridization of negatively charged nucleic acids to negatively charged capture probes. Typical salts which may be used in hybridization buffers are SSC, SSPE or PBS. Furthermore, the buffer may comprise additional ingredients such as detergents like SDS (preferably between 0.01-0.5 %), or Tween 20. Moreover, the buffer may comprise bulk DNA, which is typically added in order to reduce aspecific binding on the surface, like herring sperm DNA (hsDNA), or blocking agents like BSA. Hybridization buffers according to the present invention may also comprise ingredients to stabilize single stranded nucleic acids. An example for such an ingredient is formamide. A preferred buffer comprises 5 x SSC, 0.1% SDS and 0.1 mg/ml hsDNA.

Such buffers as well as alternative buffers, which may also be used in the context of the present invention, are known to the person skilled in the art and can be prepared according to information derivable from, e.g., Sambrook et al., Molecular Cloning: A Laboratory Manual, 2001, Cold Spring Harbor Laboratory Press.

Preferably, the hybridization reaction is performed at a temperature below the melting temperature of the complex formed between the immobilized nucleic acid molecule and the complementary oligonucleotid. More preferably, the hybridization reaction is performed at a temperature between 30 and 65°C. In a further embodiment, the hybridization as described herein above maybe combined with a washing or blocking step, which is a typical prerequisite for a subsequent hybridization reaction with specifically binding oligonucleotides. In contrast, oligonucleotides in accordance with the present invention may be shorter than specifically binding oligonucleotides and can, hence, be used at lower temperature, e.g. during necessary washing and blocking steps in the initial phase of the hybridization reaction. Oligonucleotides may accordingly be used in corresponding washing or blocking buffers. Such a procedure may save time and costs, since no additional step or extra set of buffers is necessary.

Furthermore, during a prehybridization step for the specific detection reaction at a temperature above the melting temperature of the complex formed between the immobilized nucleic acid molecule and the complementary oligonucleotide, e.g. at 50°C, the complex between said immobilized nucleic acid molecule and the complementary test olignucleotide according to the present invention may be resolved. The test oligonucleoties may accordingly be removed from the support material right in time in order to allow for an efficient hybridization reaction with the specifically binding probes.

Test oligonucleotides of the present invention, which have been removed from the support material during the prehybridization step as described herein above, may in accordance with another preferred embodiment of the present invention be reused for a subsequent interaction or control reaction in accordance with the present invention. The term "reuse" relates to a repeated usage of the oligonucleotide solution of 1 to 15 times, preferably of 1 to 5 times. This is an additional advantageous aspect of the present invention, which offers the possibility to reduce costs and allow for a high throughput control scheme with a limited amount of resource input.

In a further preferred embodiment, the present invention relates to a method for analyzing nucleic acids, comprising the steps of (a) immobilizing one or more nucleic acids on a solid support via crosslinking by heat or light or via chemical immobilization, wherein each of the immobilized nucleic acids includes a stretch of nucleotides of only one basetype; (b) providing a labeled oligonucleotide complementary to the stretch of nucleotides of only one basetype, wherein said labeled oligonucleotide is capable of forming a complex with each of the immobilized nucleic acids at the stretch of nucleotides of only one basetype; (c) detecting the presence of a specific sequence complementary to the sequence outside the stretch of nucleotides of only one basetype; and (d) determining a value indicative for the interaction between said nucleic acid and the complementary oligonucleotide via the amount of labeled oligonucleotide complementary to the stretch of nucleotides of only one basetype being in complex with the immobilized nucleic acids.

A corresponding method, in particular a combination of a step (b) which provides information on the interaction between the nucleic acid and the complementary oligonucleotide and a step (c) which may lead to the detection of the presence of specific sequence complementary to the sequence outside the stretch of nucleotides of only one basetype, allow for a parallel or realtime controlling and specific use of nucleic acid molecules immobilized on a support. Such an approach may preferably be employed in any appropriate environment known to the person skilled in the art, preferably in the ambit of hospitals or other medical facilities, or in research laboratories, where a realtime quality control of specific hybridization and interaction reactions may be particularly useful. Thereby, for example failures to the immobilized nucleic acids on the support due to shipment or production problems can be detected during the specific hybridization procedure, thus allowing for an integrated and time saving quality controlling, testing and employment of immobilized nucleic acids.

There is no strict sequential or chronological order to the steps of the method for testing nucleic acids of the present invention, provided immobilization step (a), which is a prerequisite for subsequent interaction and detection steps, is carried out first. Importantly, the steps (b) and (c) may be carried out in the order (b) first and (c) second or vice versa (c) first and (b) second. The same applies to additional steps of the method, e.g. detection or imaging steps, which be used in appropriate sequences according to the sequence of the means steps (a) and (b).

Furthermore, steps (a), (b), (c) and (d) of the method for testing nucleic acids of the present invention may be carried out without any time- or time interval-coherence between the steps, i.e. there maybe any suitable time interval between steps (a) and/or (b) and/or (c) and/or (d). The term "interval of time" relates to any suitable period of time. For instance, step (b) may be performed after a time interval of seconds, minutes, hours, days, weeks, months or even years after the performance of step (a). The same applies to step (c) with respect to step (a) and step (b) and to step (d) with respect to step (a), (b) and (c).

In a preferred embodiment, step (a) of the method for analyzing nucleic acids as defined herein above may be carried out at a different point in time than steps (b), (c) or (d). For instance the immobilization of step (a) may be carried out hours, days, weeks, months or even years before testing and/or specific controlling steps like step (b), (c) or (d) are carried out. Preferably, steps (b) to (d) are carried out after one hour to 12 months after step (a) has been initiated or terminated. A preferred "interval of time" between steps (b) and/or (c) and/or (d) may be a period of time between 1 to 60 minutes, more preferably a period of time of 1 to 30 minutes.

In a particularly preferred embodiment, steps (b) and (c) of the method for analyzing nucleic acids are carried out simultaneously.

The steps (a), (b) and (d) as mentioned above may correspond to a method for testing nucleic acids on a support as defined herein above. The step (c) maybe a specific hybridization or interaction step, which allows the complexing of complementary nucleic acid molecules, preferably of molecules which match at a percentage of between 55% to 100%, more preferably between 70% to 100%, between 80% to 100%, between 85% to 100%, and even more preferably at 90% 91%, 92%, 93%, 94%, 95%, 96%, 97 %, 98%, 99% or 100%. These values may refer to an overall matching calculated throughout the entire molecule or a local matching over the region of specific complementarity, e.g. region B as defined herein above. Means and methods for carrying out a specific hybridization and for calculating the percentage of mismatches are known to the person skilled in the art and may be derived, for example, from Sambrook et al., Molecular Cloning: A Laboratory Manual, 2001, Cold Spring Harbor Laboratory Press. Preferably, the buffer solutions and further ingredients as mentioned herein above or in the examples may be used.

For steps (b), (c) and/or (d) identical buffer conditions may be used or different buffer conditions may be used. In case different buffer conditions are used, step (c) may be carried out after an interval of time as defined herein above or vice versa, should step (c) be carried out after step (b). Between steps (b) and (c) and/or (d) a washing step may be carried out, preferably a step which is carried out under suitable conditions that guarantee a binding and remaining of the hybridized oligonucleotides to the immobilized nucleic acids.

For the detection of interaction or hybridization of oligonucleotides to immobilized nucleic acids as mentioned in step (b) of the method for analyzing nucleic acids as defined herein above a label may be used which is optically or chemically distinguishable from a label to be used in step (c) of said method, i.e. the control interaction and specific hybridization reactions may be carried out by using two different, distinguishable labels. Suitable labels are known to the person skilled in the art and have been described herein above. Preferably, two different fluorescent labels are used.

In addition to the control of immobilized nucleic acids the present invention also provides a possibility to independently control the quality of nucleic acids immobilized on the support material by the employment of an additional, specifically binding oligonuleotide. In a preferred embodiment, the method foresees the provision of at least one labeled test oligonucleotide which is complementary to a predefined specific stretch of nucleotides outside the stretch of nucleotides of only one basetype. Furthermore said labeled oligonucleotide is capable of distinctively forming a complex with immobilized nucleic acids which comprise said specific stretch of nucleotides. Subsequently, a value indicative for the interaction between said nucleic acid and the complementary test oligonucleotide via the presence of said test oligonucleotide being in complex with the predefined specific stretch of nucleotides outside the stretch of nucleotides of only one basetype of the immobilized nucleic acids which comprise said specific stretch of nucleotides maybe determined. Such a independent testing provides a further control layer to the method as defined herein above, since now not only an interaction between the control oligo and virtually all immobilized nucleic acids can be detected, but also a specific interaction between one or more immobilized nucleic acids (depending on the number of times the specific sequence is present among the deposited nucleic acids) and a specific test oligonculeotide. Such a secondary control reaction may be carried out for one or several distinct specific sequences. The number of different specific sequences depends on the number of different probes present on the support material. In a preferred embodiment, between 0.1% and 10% of all immobilized nucleic acids may be tested in such a secondary test approach in order to obtain a statistically relevant feedback with respect to the capability of the immobilized nucleic acids to bind to specifically binding complementary oligonucleotides. Preferably, a number of 1, 2, 3, 4, 5 or 6 specific secondary control reactions are carried out. Any discrepancy between the results of the primary control approach using oligonucleotides being complementary to stretches of identical basetypes in the immobilized nucleic acid molecules and the results of the secondary control approach using specifically binding oligonucleotides may be indicative for problems being particularly related to either of these control approaches.

Preferably, such a secondary control approach may be carried out by using a label which is optically or chemically distinguishable from a label used in the primary control approach. In order to allow a differentiation between primary and secondary control approach these labels should not be located on one and the same oligonucleotide having, for example two distinct regions, one comprising a stretch of nucleotides of only one basetype and a further one comprising a specific stretch of nucleotides, since in such a setup no distinction between a binding reaction to a region comprising a stretch of nucleotides of only one basetype and a binding reaction to a region comprising a specific stretch of nucleotides can be achieved.

In addition to the method as described herein above also a kit for the application of a secondary control approach is comprised by the present invention as an additional, preferred embodiment. Such a kit may comprises some or all ingredients of a kit according to the present invention as set forth herein above and additionally at least one labeled test oligonucleotide complementary to a predefined specific stretch of nucleotides outside the stretch of nucleotides of only one basetype, wherein said labeled oligonucleotide is capable of distinctively forming a complex with immobilized nucleic acids which comprise said specific stretch of nucleotides.

The following examples and figures are provided for illustrative purposes. It is thus understood that the example and figures are not to be construed as limiting. The skilled person in the art will clearly be able to envisage further modifications of the principles laid out herein.

### EXAMPLES

### Example 1 - Control probe assay

A membrane was printed and post-processed using UV-light at a wavelength of 254 nm and a standard pre-hybridization method. An outline of the deposited nucleic acids etc. can be derived from Fig. 2A.

After post-processing, an image of the membrane only shows the labeled spots (see Fig. 2B). Subsequently, the membrane was incubated with a labeled A16 oligonucleotide for a time period of one hour at a temperature of 50 °C. As label Cy5 was used. The hybridization buffer was 5 x SSC, 0.1% SDS, 0.1 mg/ml herring sperm DNA. Hybridization was done at 50°C during 1 hour. After hybridization, a short rinse with 2 x SSC and 0.1% SDS was carried out. Subsequently, the membrane was dried and the array was imaged.

As can be derived from Fig. 2C, the hybridization spots are clearly visible. This means that in all areas, in which DNA was deposited and immobilized DNA is present. Furthermore, the deposited and immobilized DNA is able to hybridize with an adenine control hybridization probe.

This proves that a method based on the use of a control probe complementary to a stretch of nucleotides of only one basetype can effectively be employed for quality controlling measures, e.g. in manufacturing processes of the membranes.

### Example 2 - Testing of membrane for non-disruptiveness of method

In order to prove that the control method is non-disruptive, the membrane used in Example 1, i.e. in a control and test hybridization approach as depicted in Figs. 2B to 2C, was subsequently heated up to remove the control probe.

The image of the membrane directly after heating up in order to remove all the control oligonucleotides from the capture probe spots shows that the hybridization spots no longer comprise any signal (see Fig. 2D).

To prove that control method as described in Example 1 does not harm the sequence of the immobilized nucleic acid, which is to be used for specific hybridization and binding of a specific oligonucleotide, the membrane was subsequently incubated with 10 nM of a labeled antisense molecule, which is complimentary to the DNA deposited on spot # 4. The membrane was incubated for a time period of one hour at a temperature of 50 °C during 1 hour. The hybridization buffer was 5 x SSC, 0.1% SDS, 0.1 mg/ml herring sperm DNA. After hybridization, a short rinse with 2 x SSC and 0.1% SDS was performed. Subsequently, the membrane was dried and the array was imaged.

Hybridization signals can clearly be seen after the incubation of the membrane (see Fig. 3; marked in thick squares are the spots which show a signal after hybridization with the labeled antisense oligonucleotide, corresponding to spot # 4 as indicated in Fig. 2A.).

This result allows the conclusion that the immobilized nucleic acid molecule was not damaged during the primary control step and can still be bound by a specific antisense oligonucleotide.

### Example 3 - Recovery testing and sensitivity testing of nucleic acids comprising a T-tail

The sensitivity, i.e. the number of captured analytes per unit of time, was tested in a real-time hybridization assay. Nytran N or Nytran SPC nylon membranes were used for the experiments.

The assay was carried out with capture oligonucleotides (i.e. deposited nucleic acid molecules to be immobilized) comprising either no T-tail or a T16-tail, i.e. a stretch of 16 thymidines. These experiments were done in a flow cell, which is a device into which the membrane is clamped and the hybridization fluid is pumped through the membrane. In figure 4A, on the X-axis, the cycle number is depicted, which is an equivalent for the time (1 cycle takes 1 minute). Hybridization was done with complementary DNA. The hybridization buffer was 5 x SSC, 0.1% SDS, 0.1 mg/ml herring sperm DNA. The temperature was set at 50 ° C.

As can be derived from Fig. 4A the oligonucleotides comprising a T16-tail show increased hybridization signals, which are attributed to higher recovery. The recovery is the ratio between immobilized oligonucleotides and deposited oligonucleotides.

The experiment shows that the recovery and, in consequence, the sensitivity increases with increasing number of nucleotides of only one basetype within the capture molecule.

A normalization of the results, as can be derived from Fig. 4B, which shows an averaged recovery rate of deposited capture oligonucleotides comprising T or A nucleotides as a function of the base type (T or A) and the number of bases (2, 4, 8, 16 or 32), makes clear that the recovery can be increased by a factor of 3-4 when the number of nucleotides of only one basetype within the capture oligonucleotide, i.e. the number of Ts, is increased from 2 to 32.

### Example 4 - Specificity testing of immobilized nucleic acids

The specificity of immobilized nucleic acids, i.e. the ability to distinguish between matching and mismatching targets, was tested in a binding assay.

The assay was performed with immobilized nucleic acids (capture probes) comprising 0, 4 or 16 T's. Different capture probes were used that contained perfect match, single mismatch ((AG)mut)and double mismatches((AAGG)mut). Hybridization was done using PCR product. Hybridization was done in a flow cell, which is a device into which the membrane is clamped and the hybridization fluid is pumped through the membrane. The temperature was set at 50 ° C. Hybridization was done for one hour. After hybridization, the buffer was changed to 2 x SSC and the temperature was increased with 1 ° C/min in order to make a melting curve and to assess specificity.

As can be derived from Fig. 5, which depicts de-binding curves of the complementary, single mismatch and double mismatch hybrids for the different capture probes, an increasing selectivity was obtained due to increased melting temperatures of the complementary probes as compared to mismatch probes.

### Example 5 - Effect of abasic sites on hybridization intensity

The effect of abasic sites on hybridization intensity of nucleic acid molecules from DNA with a perfect match and single mismatch ((AG)mut)and double mismatches((AAGG)mut was tested in a binding assay. The capture probe comprised 0, 2, 4 or 8 abasic sites. The binding assay was carried out with complementary target oligonucleotides on NytranN nylon membranes.

As can be derived from Fig. 6, the hybridization intensity increased with an increasing number of abasic sites in all tested scenarios, i.e. the hybridization with complementary target oligonucleotides, mismatch target oligonucleotides or double mismatch target oligonucleotides. The effect is attributable to a more efficient separation of the sequences used for specific immobilization and specific hybridization, which decreases unspecific hybridization.

## Claims

1. A method for testing nucleic acids on a support, comprising the steps of:
(a) immobilizing one or more nucleic acids on a solid support allowing for an accurate and trackable positioning of nucleic acids on the support material via crosslinking by heat or light or via chemical immobilization, wherein each of the immobilized nucleic acids includes a stretch of nucleotides of only one basetype;
(b) providing a labeled oligonucleotide complementary to the stretch of nucleotides of only one basetype, wherein said labeled oligonucleotide is capable of forming a complex with each of the immobilized nucleic acids at the stretch of nucleotides of only one basetype; and
(c) determining a value indicative for the interaction between said nucleic acid and the complementary oligonucleotide via the amount of labeled oligonucleotide being in complex with the immobilized nucleic acid.

2. A kit for testing nucleic acids on a support, comprising:
(a) an array of nucleic acids immobilized on a solid support allowing for an accurate and trackable positioning of nucleic acids on the support material via crosslinking by heat or light or via chemical immobilization, wherein each of the immobilized nucleic acids includes a stretch of nucleotides of only one basetype; and
(b) a labeled oligonucleotide complementary to the stretch of nucleotides of only one basetype, wherein said labeled oligonucleotide is capable of forming a complex with each of the immobilized nucleic acids at the stretch of nucleotides of only one basetype.

3. Use of a labeled oligonucleotide complementary to a stretch of nucleotides of only one basetype for testing the interaction between the complementary oligonucleotide and nucleic acids immobilized on a solid support allowing for an accurate and trackable positioning of nucleic acids on the support material via crosslinking by heat or light or via chemical immobilization, wherein each of the immobilized nucleic acids includes a stretch of nucleotides of only one basetype and wherein said labeled oligonucleotide is capable of forming a complex with each of the immobilized nucleic acids at said stretch of nucleotides of only one basetype.

4. The use of claim 3, wherein said testing the interaction between the complementary oligonucleotide and nucleic acids comprises the determination of a value indicative for the amount of labeled oligonucleotide being in complex with said immobilized nucleic acid.

5. The method of claim 1, the kit of claim 2 or the use of claim 3 or 4, wherein said nucleic acid is a single-stranded DNA, RNA, PNA, CNA, HNA, LNA or ANA; an oligonucleotide thereof; or any combination thereof.

6. The method of claim 1, the kit of claim 2 or the use of claim 3 or 4, wherein said stretch of nucleotides of only one basetype is a stretch of thymines, uracils or guanines.

7. The method, kit or use of claim 6, wherein said stretch of nucleotides of only one basetype has a length up to 100 nucleotides, preferably of 16 nucleotides.

8. The method of claim 1, the kit of claim 2 or the use of claim 3 or 4, wherein said crosslinking is crosslinking by light performed at a wavelength of 200-300 nm, preferably at 254 nm, or of 300-500 nm, preferably at 365 nm and using an amount of energy ranging from 0.1 Joule/cm² to 10 Joule/cm².

9. The method of claim 1, the kit of claim 2 or the use of claim 3 or 4, wherein said chemical immobilization is a coupling between an amine-modified nucleic acid and a corresponding functional group on the solid support, wherein said functional group is preferably an epoxy, aldehyde, carboxylate or NHS group.

10. The method of claim 1, the kit of claim 2 or the use of claim 3 or 4, wherein said stretch of nucleotides of only one basetype is located at the 3' or 5' terminus of said nucleic acid.

11. The method of any one of claims 1, 5, 6, 9 or 10, the kit of any one of claims 2, 5, 6, 9 or 10 or the use of any one of claim 3 to 6, 9 or 10, wherein said nucleic acid is represented by the following formula:
5'-Yₙ-Xₘ-Bᵣ-Xₚ-Z_{q}-3'
with Y and Z being stretches of nucleotides of only one basetype, wherein Y and Z can be of the same or of a different basetype; X being a spacer, preferably composed of abasic nucleotides; B being a sequence of more than one basetype and n, m, r, p and q being the numbers of nucleotides in the nucleic acid, wherein the following conditions apply: n, m, p, q, r >1; n, m, r > 1 and p, q = 0; p, q, r >1 and n, m = 0; n, q, r > 1 and m, p = 0; n, r > 1 and m, p, q = 0; q, r > 1 and n, m, p = 0.

12. The method of claim 1, the kit of claim 2 or the use of claim 3 or 4, wherein said labeled oligonucleotide comprises a fluorescent, radioactive or chemiluminescent label.

13. The method of claim 1, the kit of claim 2 or the use of claim 3 or 4, wherein said solid support comprises amine-functionalized groups, preferably primary or secondary amines and more preferably is a porous substrate like nylon or a non-porous substrate like glass, poly-L-lysine coated material, nitrocellulose, polystyrene, cyclic olefin copolymer (COC), cyclic olefin polymer (COP), polypropylene, polyethylene or polycarbonate.

14. The method of claim 1 or 13, wherein said labeled oligonucleotide complementary to the stretch of nucleotides of only one basetype is obtained for re-use in a further step (d) by increasing the temperature above the melting temperature of said labeled oligonucleotide.

15. A method for analyzing nucleic acids, comprising the steps of:
(a) immobilizing one or more nucleic acids on a solid support allowing for an accurate and trackable positioning of nucleic acids on the support material via crosslinking by heat or light or via chemical immobilization, wherein each of the immobilized nucleic acids includes a stretch of nucleotides of only one basetype;
(b) providing a labeled oligonucleotide complementary to the stretch of nucleotides of only one basetype, wherein said labeled oligonucleotide is capable of forming a complex with each of the immobilized nucleic acids at the stretch of nucleotides of only one basetype;
(c) detecting the presence of a specific sequence complementary to the sequence outside the stretch of nucleotides of only one basetype; and
(d) determining a value indicative for the interaction between said nucleic acid and the complementary oligonucleotide via the amount of labeled oligonucleotide complementary to the stretch of nucleotides of only one basetype being in complex with the immobilized nucleic acids.

16. The method of any one of claims 1, 13 or 14, wherein the quality of said immobilized nucleic acids is additionally tested by the further steps of
(i) providing at least one labeled test oligonucleotide complementary to a predefined specific stretch of nucleotides outside the stretch of nucleotides of only one basetype, wherein said labeled oligonucleotide is capable of distinctively forming a complex with immobilized nucleic acids which comprise said specific stretch of nucleotides; and
(ii) determining a value indicative for the interaction between said nucleic acid and the complementary oligonucleotide via the presence of said test oligonucleotide being in complex with the predefined specific stretch of nucleotides outside the stretch of nucleotides of only one basetype of the immobilized nucleic acids which comprise said specific stretch of nucleotides.

17. The kit for testing nucleic acids on a support of claim 2, which additionally comprises
(c) at least one labeled test oligonucleotide complementary to a predefined specific stretch of nucleotides outside the stretch of nucleotides of only one basetype, wherein said labeled oligonucleotide is capable of distinctively forming a complex with immobilized nucleic acids which comprise said specific stretch of nucleotides.

18. The method of claim 15 or 16 or the kit of claim 17, wherein said labeled test oligonucleotide complementary to a predefined stretch of nucleotides outside the stretch of nucleotides of only one basetype is labeled with a label which is optically or chemically distinguishable from the label of the labeled oligonucleotide complementary to the stretch of nucleotides of only one basetype.

## Patentansprüche

1. Verfahren zum Testen von Nukleinsäuren auf einem Träger, die Schritte umfassend:
(a) Immobilisieren einer oder mehrerer Nukleinsäuren auf einem festen Träger, zum Erlauben einer genauen und verfolgbaren Positionierung von Nukleinsäuren auf dem Trägermaterial, mittels Vernetzung durch Wärme oder Licht oder mittels chemischer Immobilisierung, wobei jede der immobilisierten Nukleinsäuren einen Abschnitt von Nukleotiden mit nur einem Grundtyp enthält;
(b) Bereitstellen eines markierten Oligonukleotids ergänzend zu dem Abschnitt von Nukleotiden mit nur einem Grundtyp, wobei das besagte markierte Oligonukleotid imstande ist, einen Komplex mit jeder der immobilisierten Nukleinsäuren auf dem Abschnitt von Nukleotiden mit nur einem Grundtyp zu bilden; und
(c) Bestimmen eines Wertes als Hinweis auf die Interaktion zwischen der besagten Nukleinsäure und dem ergänzenden Oligonukleotid anhand der Menge des markierten Oligonukleotids, das sich im Komplex mit der immobilisierten Nukleinsäure befindet.

2. Kit zum Testen von Nukleinsäuren auf einem Träger, umfassend:
(a) eine Anordnung von Nukleinsäuren, die auf einem festen Träger immobilisiert sind, zum Erlauben einer genauen und verfolgbaren Positionierung von Nukleinsäuren auf dem Trägermaterial, mittels Vernetzung durch Wärme oder Licht oder mittels chemischer Immobilisierung, wobei jede der immobilisierten Nukleinsäuren einen Abschnitt von Nukleotiden mit nur einem Grundtyp enthält; und
(b) ein markiertes Oligonukleotid ergänzend zu dem Abschnitt von Nukleotiden mit nur einem Grundtyp, wobei das besagte markierte Oligonukleotid imstande ist, einen Komplex mit jeder der immobilisierten Nukleinsäuren auf dem Abschnitt von Nukleotiden mit nur einem Grundtyp zu bilden.

3. Verwendung eines markierten Oligonukleotids ergänzend zu einem Abschnitt von Nukleotiden mit nur einem Grundtyp zum Testen der Interaktion zwischen dem ergänzenden Oligonukleotid und Nukleinsäuren, die auf einem festen Träger immobilisiert sind, zum Erlauben einer genauen und verfolgbaren Positionierung von Nukleinsäuren auf dem Trägermaterial, mittels Vernetzung durch Wärme oder Licht oder mittels chemischer Immobilisierung, wobei jede der immobilisierten Nukleinsäuren einen Abschnitt von Nukleotiden mit nur einem Grundtyp enthält, und wobei das besagte markierte Oligonukleotid imstande ist, einen Komplex mit jeder der immobilisierten Nukleinsäuren auf dem Abschnitt von Nukleotiden mit nur einem Grundtyp zu bilden.

4. Verwendung nach Anspruch 3, wobei das besagte Testen der Interaktion zwischen dem ergänzenden Oligonukleotid und Nukleinsäuren die Bestimmung eines Wertes als Hinweis auf die Menge des markierten Oligonukleotids umfasst, das sich im Komplex mit der besagten immobilisierten Nukleinsäure befindet.

5. Verfahren nach Anspruch 1, Kit nach Anspruch 2 oder Verwendung nach Anspruch 3 oder 4, wobei die besagte Nukleinsäure eine einsträngige DNA, RNA, PNA, CNA, HNA, LNA oder ANA ist; ein Oligonukleotid davon; oder irgendeine Kombination davon.

6. Verfahren nach Anspruch 1, Kit nach Anspruch 2 oder Verwendung nach Anspruch 3 oder 4, wobei der besagte Abschnitt von Nukleotiden mit nur einem Grundtyp ein Abschnitt von Thyminen, Uracilen oder Guaninen ist.

7. Verfahren, Kit oder Verwendung nach Anspruch 6, wobei der besagte Abschnitt von Nukleotiden mit nur einem Grundtyp eine Länge von bis zu 100 Nukleotiden, vorzugsweise von 16 Nukleotiden aufweist.

8. Verfahren nach Anspruch 1, Kit nach Anspruch 2 oder Verwendung nach Anspruch 3 oder 4, wobei die besagte Vernetzung eine Vernetzung durch Licht ist, die mit einer Wellenlänge von 200-300 nm, vorzugsweise mit 254 nm, oder mit 300-500 nm, vorzugsweise mit 365 nm und unter Verwendung einer Menge an Energie im Bereich von 0,1 Joule/cm² bis 10 Joule/cm² durchgeführt wird.

9. Verfahren nach Anspruch 1, Kit nach Anspruch 2 oder Verwendung nach Anspruch 3 oder 4, wobei die besagte chemische Immobilisierung eine Verbindung zwischen einer aminmodifizierten Nukleinsäure und einer entsprechenden funktionellen Gruppe des festen Trägers ist, wobei die besagte funktionelle Gruppe vorzugsweise eine Epoxy-, Aldehyd-, Carboxylat- oder NHS-Gruppe ist.

10. Verfahren nach Anspruch 1, Kit nach Anspruch 2 oder Verwendung nach Anspruch 3 oder 4, wobei sich der besagte Abschnitt von Nukleotiden mit nur einem Grundtyp am 3'- oder 5'-Ende der besagten Nukleinsäure befindet.

11. Verfahren nach einem der Ansprüche 1, 5, 6, 9 oder 10, Kit nach einem der Ansprüche 2, 5, 6, 9 oder 10 oder Verwendung nach einem der Ansprüche 3 bis 6, 9 oder 10, wobei die besagte Nukleinsäure durch die folgende Formel dargestellt ist:
5'-Yₙ-Xₘ-Bᵣ-Xₚ-Z_{q}-3'
wobei Y und Z Abschnitte von Nukleotiden mit nur einem Grundtyp sind, wobei Y und Z denselben oder einen unterschiedlichen Grundtyp aufweisen können; wobei X ein Spacer ist, der sich vorzugsweise aus abasischen Nukleotiden zusammensetzt; wobei B eine Sequenz aus mehr als einem Grundtyp ist, und n, m, r, p und q die Anzahl an Nukleotiden in der Nukleinsäure sind, wobei die folgenden Bedingungen zur Anwendung kommen: n, m, p, q, r> 1; n, m, r> 1 und p, q = 0; p, q, r> 1 und n, m = 0; n, q, r > 1 und m, p = 0; n, r > 1 und m, p, q = 0; q, r > 1 und n, m, p = 0.

12. Verfahren nach Anspruch 1, Kit nach Anspruch 2 oder Verwendung nach Anspruch 3 oder 4, wobei das besagte markierte Oligonukleotid eine fluoreszierende, radioaktive oder chemilumineszierende Markierung umfasst.

13. Verfahren nach Anspruch 1, Kit nach Anspruch 2 oder Verwendung nach Anspruch 3 oder 4, wobei der feste Träger aminofunktionalisierte Gruppen, vorzugsweise primäre oder sekundäre Amine umfasst und am besten ein poröses Substrat wie Nylon oder ein nicht poröses Substrat wie Glas, ein mit Poly-L-Lysin beschichtetes Material, Nitrocellulose, Polystryrol, zyklisches Olefin-Copolymer (COC), zyklisches Olefin-Polymer (COP), Polypropylen, Polyethylen oder Polycarbonat ist.

14. Verfahren nach Anspruch 1 oder 13, wobei man das besagte markierte Oligonukleotid ergänzend zu dem Abschnitt von Nukleotiden mit nur einem Grundtyp zur Wiederverwendung in einem weiteren Schritt (d) durch Erhöhen der Temperatur über die Schmelztemperatur des besagten markierten Oligonukleotids hinaus erhält.

15. Verfahren zum Analysieren von Nukleinsäuren, die Schritte umfassend:
(a) Immobilisieren einer oder mehrerer Nukleinsäuren auf einem festen Träger, zum Erlauben einer genauen und verfolgbaren Positionierung von Nukleinsäuren auf dem Trägermaterial, mittels Vernetzung durch Wärme oder Licht oder mittels chemischer Immobilisierung, wobei jede der immobilisierten Nukleinsäuren einen Abschnitt von Nukleotiden mit nur einem Grundtyp enthält;
(b) Bereitstellen eines markierten Oligonukleotids ergänzend zu dem Abschnitt von Nukleotiden mit nur einem Grundtyp, wobei das besagte markierte Oligonukleotid imstande ist, einen Komplex mit jeder der immobilisierten Nukleinsäuren auf dem Abschnitt von Nukleotiden mit nur einem Grundtyp zu bilden;
(c) Detektieren der Präsenz einer spezifischen Sequenz ergänzend zu der Sequenz außerhalb des Abschnitts von Nukleotiden mit nur einem Grundtyp; und
(d) Bestimmen eines Wertes als Hinweis auf die Interaktion zwischen der besagten Nukleinsäure und dem ergänzenden Oligonukleotid anhand der Menge des markierten Oligonukleotids, das sich im Komplex mit der immobilisierten Nukleinsäure befindet.

16. Verfahren nach einem der Ansprüche 1, 13 oder 14, wobei die Qualität der besagten immobilisierten Nukleinsäuren zusätzlich getestet wird, durch die weiteren Schritte
(i) Bereitstellen zumindest eines markierten Test-Oligonukleotids ergänzend zu einem vordefinierten spezifischen Abschnitt von Nukleotiden außerhalb des Abschnitts von Nukleotiden mit nur einem Grundtyp, wobei das besagte markierte Oligonukleotid imstande ist, unterscheidbar einen Komplex mit immobilisierten Nukleinsäuren zu bilden, der den besagten spezifischen Abschnitt von Nukleotiden umfasst; und
(ii) Bestimmen eines Wertes als Hinweis auf die Interaktion zwischen der besagten Nukleinsäure und dem ergänzenden Oligonukleotid anhand der Präsenz des besagten Test-Oligonukleotids, das in Komplex mit dem vordefinierten spezifischen Abschnitt von Nukleotiden außerhalb des Abschnitts von Nukleotiden mit nur einem Grundtyp der immobilisierten Nukleinsäuren ist, die den besagten spezifischen Abschnitt von Nukleotiden umfassen.

17. Kit zum Testen von Nukleinsäuren auf einem Träger nach Anspruch 2, der zusätzlich umfasst:
(c) Zumindest ein markiertes Test-Oligonukleotid ergänzend zu einem vordefinierten spezifischen Abschnitt von Nukleotiden außerhalb des Abschnitts von Nukleotiden mit nur einem Grundtyp, wobei das besagte markierte Oligonukleotid imstande ist, unterscheidbar einen Komplex mit immobilisierten Nukleinsäuren zu bilden, der den besagten spezifischen Abschnitt von Nukleotiden umfasst.

18. Verfahren nach Anspruch 15 oder 16, oder Kit nach Anspruch 17, wobei das besagte markierte Test-Oligonukleotid ergänzend zu einem vordefinierten spezifischen Abschnitt von Nukleotiden außerhalb des Abschnitts von Nukleotiden mit nur einem Grundtyp, mit einer Markierung markiert ist, die optisch oder chemisch von der Markierung des markierten Oligonukleotids ergänzend zu dem Abschnitt von Nukleotiden mit nur einem Grundtyp unterscheidbar ist.

## Revendications

1. Procédé de test d'acides nucléiques sur un support, comprenant les étapes de :
(a) immobilisation d'un ou plusieurs acides nucléiques sur un support solide permettant un positionnement précis et localisable d'acides nucléiques sur le matériau de support via réticulation par la chaleur ou la lumière ou via immobilisation chimique, dans lequel chacun des acides nucléiques immobilisés comporte un prolongement de nucléotides d'un seul type de base ;
(b) fourniture d'un oligonucléotide marqué complémentaire au prolongement de nucléotides d'un seul type de base, dans lequel ledit oligonucléotide marqué est capable de former un complexe avec chacun des acides nucléiques immobilisés sur le prolongement des nucléotides d'un seul type de base ; et
(c) détermination d'une valeur indicative de l'interaction entre ledit acide nucléique et l'oligonucléotide complémentaire via la quantité d'oligonucléotide marqué qui est complexé à l'acide nucléique immobilisé.

2. Kit de test d'acides nucléiques sur un support, comprenant :
(a) une série d'acides nucléiques immobilisés sur un support solide permettant un positionnement précis et localisable d'acides nucléiques sur le matériau de support via réticulation par la chaleur ou la lumière ou via immobilisation chimique, dans lequel chacun des acides nucléiques immobilisés comporte un prolongement de nucléotides d'un seul type de base ; et
(b) un oligonucléotide marqué complémentaire au prolongement de nucléotides d'un seul type de base, dans lequel ledit oligonucléotide marqué est capable de former un complexe avec chacun des acides nucléiques immobilisés sur le prolongement de nucléotides d'un seul type de base.

3. Utilisation d'un oligonucléotide marqué complémentaire à un prolongement de nucléotides d'un seul type de base pour tester l'interaction entre l'oligonucléotide complémentaire et les acides nucléiques immobilisés sur un support solide permettant un positionnement précis et localisable d'acides nucléiques sur le matériau de support via une réticulation par la chaleur ou la lumière ou via immobilisation chimique, dans laquelle chacun des acides nucléiques immobilisés comporte un prolongement de nucléotides d'un seul type de base et dans lequel ledit oligonucléotide marqué est capable de former un complexe avec chacun des acides nucléiques immobilisés sur ledit prolongement de nucléotides d'un seul type de base.

4. Utilisation selon la revendication 3, dans laquelle ledit test de l'interaction entre l'oligonucléotide complémentaire et les acides nucléiques comprend la détermination d'une valeur indicative de la quantité d'oligonucléotide marqué qui est complexé avec ledit acide nucléique immobilisé.

5. Procédé selon la revendication 1, kit selon la revendication 2 ou utilisation selon la revendication 3 ou 4, dans lesquels ledit acide nucléique est un ADN à brin simple, ARN, PNA, CNA, HNA, LNA ou ANA ; un oligonucléotide de ceux-ci ; ou toute combinaison de ceux-ci.

6. Procédé selon la revendication 1, kit selon la revendication 2 ou utilisation selon la revendication 3 ou 4, dans lesquels ledit prolongement de nucléotides d'un seul type de base est un prolongement de thymines, d'uraciles ou de guanines.

7. Procédé, kit ou utilisation selon la revendication 6, dans lesquels ledit prolongement de nucléotides d'un seul type de base a une longueur allant jusqu'à 100 nucléotides, de préférence, de 16 nucléotides.

8. Procédé selon la revendication 1, kit selon la revendication 2 ou utilisation selon la revendication 3 ou 4, dans lesquels ladite réticulation est une réticulation par la lumière réalisée à une longueur d'ondes de 200 à 300 nm, de préférence à 254 nm, ou de 300 à 500 nm, de préférence à 365 nm et utilisant une quantité d'énergie allant de 0,1 joule/cm² à 10 joules/cm².

9. Procédé selon la revendication 1, kit selon la revendication 2 ou utilisation selon la revendication 3 ou 4, dans lesquels ladite immobilisation chimique est un couplage entre un acide nucléique modifié par une amine et un groupe fonctionnel correspondant sur le support solide, dans lequel ledit groupe fonctionnel est de préférence un groupe époxy, aldéhyde, carboxylate ou NHS.

10. Procédé selon la revendication 1, kit selon la revendication 2 ou utilisation selon la revendication 3 ou 4, dans lesquels ledit prolongement de nucléotides d'un seul type de base est situé à la terminaison 3' ou 5' dudit acide nucléique.

11. Procédé selon l'une quelconque des revendications 1, 5, 6, 9 ou 10, kit selon l'une quelconque des revendications 2, 5, 6, 9 ou 10 ou utilisation selon l'une quelconque des revendications 3 à 6, 9 ou 10, dans lesquels ledit acide nucléique est représenté par la formule suivante :
5'-Yₙ-Xₘ-B₁-Xₚ-Z_{q}-3'
Y et Z étant des prolongements de nucléotides d'un seul type de base, dans lesquels Y et Z peuvent être du même type de base ou d'un type de base différent ; X étant un segment d'espacement, de préférence composé de nucléotides abasiques ; B étant une séquence de plus d'un type de base et n, m, r, p et q étant les nombres de nucléotides dans l'acide nucléique, dans lesquels les conditions suivantes s'appliquent : n, m, p, q, r > 1 ; n, m, r > 1 et p, q = 0 ; p, q, r > 1 et n, m = 0 ; n, q, r > 1 et m, p = 0 ; n, r > 1 et m, p, q = 0 ; q, r > 1 et n, m, p = 0.

12. Procédé selon la revendication 1, kit selon la revendication 2 ou utilisation selon la revendication 3 ou 4, dans lesquels ledit oligonucléotide marqué comprend un marqueur fluorescent, radioactif ou chimioluminescent.

13. Procédé selon la revendication 1, kit selon la revendication 2 ou utilisation selon la revendication 3 ou 4, dans lesquels ledit support solide comprend des groupes fonctionnalisés par une amine, de préférence des amines primaires ou secondaires et de manière davantage préférée, est un substrat poreux de type nylon ou un substrat non poreux tel que le verre, un matériau revêtu de poly-L-lysine, la nitrocellulose, le polystyrène, un copolymère d'oléfine cyclique (COC), un polymère d'oléfine cyclique (COP), un polypropylène, un polyéthylène ou un polycarbonate.

14. Procédé selon la revendication 1 ou 13, dans lequel ledit oligonucléotide marqué complémentaire au prolongement de nucléotides d'un seul type de base est obtenu pour une réutilisation dans une autre étape (d) en augmentant la température au-dessus de la température de fusion dudit oligonucléotide marqué.

15. Procédé d'analyse d'acides nucléiques, comprenant les étapes de :
(a) immobilisation d'un ou plusieurs acides nucléiques sur un support solide permettant un positionnement précis et localisable d'acides nucléiques sur le matériau de support via réticulation par la chaleur ou la lumière ou via immobilisation chimique, dans lequel chacun des acides nucléiques immobilisés comporte un prolongement de nucléotides d'un seul type de base ;
(b) fourniture d'un oligonucléotide marqué complémentaire au prolongement de nucléotides d'un seul type de base, dans lequel ledit oligonucléotide marqué est capable de former un complexe avec chacun des acides nucléiques immobilisés sur le prolongement des nucléotides d'un seul type de base ; et
(c) détection de la présence d'une séquence spécifique complémentaire à la séquence hors du prolongement de nucléotides d'un seul type de base ; et
(d) détermination d'une valeur indicative de l'interaction entre ledit acide nucléique et l'oligonucléotide complémentaire via la quantité d'oligonucléotide marqué complémentaire au prolongement de nucléotides d'un seul type de base qui est complexé aux acides nucléiques immobilisés.

16. Procédé selon l'une quelconque des revendications 1, 13 ou 14, dans lequel la qualité desdits acides nucléiques immobilisés est testée de plus par les autres étapes de
(i) fourniture d'au moins un oligonucléotide de test marqué complémentaire à un prolongement spécifique prédéfini de nucléotides hors du prolongement de nucléotides d'un seul type de base, dans lequel ledit oligonucléotide marqué est capable de former de façon distincte un complexe avec des acides nucléiques immobilisés qui comprennent ledit prolongement spécifique de nucléotides ; et
(ii) détermination d'une valeur indicative de l'interaction entre ledit acide nucléique et l'oligonucléotide complémentaire via la présence dudit oligonucléotide de test qui est complexé avec le prolongement spécifique prédéfini de nucléotides hors du prolongement de nucléotides d'un seul type de base des acides nucléiques immobilisés qui comprennent ledit prolongement spécifique de nucléotides.

17. Kit de test d'acides nucléiques sur un support selon la revendication 2, qui comprend de plus
(c) au moins un oligonucléotide de test marqué complémentaire à un prolongement spécifique prédéfini de nucléotides hors du prolongement de nucléotides d'un seul type de base, dans lequel ledit oligonucléotide marqué est capable de former de façon distincte un complexe avec des acides nucléiques immobilisés qui comprennent ledit prolongement spécifique de nucléotides.

18. Procédé selon la revendication 15 ou 16 ou kit selon la revendication 17, dans lequel ledit oligonucléotide de test marqué complémentaire à un prolongement prédéfini de nucléotides hors du prolongement de nucléotides d'un seul type de base est marqué avec un marqueur qui peut être distingué optiquement ou chimiquement du marqueur de l'oligonucléotide complémentaire marqué au prolongement de nucléotides d'un seul type de base.
